(19) Europäisches Patentamt
European Patent Office
Office européen des brevets

(11) EP 0 850 946 A1

(12) **EUROPÄISCHE PATENTANMELDUNG**

(43) Veröffentlichungstag: **01.07.1998 Patentblatt 1998/27**

(21) Anmeldenummer: **97810988.2**

(22) Anmeldetag: **16.12.1997**

(51) Int Cl.[6]: **C07F 9/6571**, C08K 5/5393, C07C 39/23, C07C 39/205, C07D 309/22, C07D 335/02

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **24.12.1996 CH 3198/96**

(71) Anmelder: **Ciba Specialty Chemicals Holding Inc.**
**4057 Basel (CH)**

(72) Erfinder:
- **Nesvadba, Peter**
  **1723 Marly (CH)**
- **Dubs, Paul**
  **1700 Fribourg (CH)**

(54) **Cyclische Phosphinsäurederivate als Stabilisatoren**

(57) Es werden neue Verbindungen der Formel I

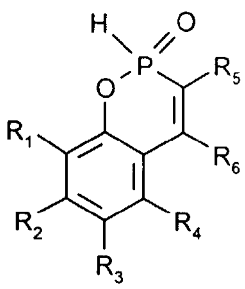

(I)

worin die allgemeinen Symbole wie in Anspruch 1 definiert sind, als Stabilisatoren für organische Materialien gegen oxidativen, thermischen oder lichtinduzierten Abbau beschrieben.

EP 0 850 946 A1

## Beschreibung

Die vorliegende Erfindung betrifft neue cyclische Phosphinsäurederivate, Zusammensetzungen, enthaltend ein organisches Material, bevorzugt ein Polymer, und die neuen cyclischen Phosphinsäurederivate, sowie die Verwendung derselben zum Stabilisieren von organischen Materialien gegen oxidativen, thermischen oder lichtinduzierten Abbau.

Organische Phosphite, Phosphonite und Phosphoramide sind in der Technik as Costabilisatoren, sekundäre Antioxidantien und Verarbeitungsstabilisatoren, unter anderem für Polyolefine, bekannt. Beispiele für solche bekannten Phosphitstabilisatoren finden sich in R. Gächter/H. Müller (Ed.), Plastics Additives Handbook, 3rd Ed., Seite 47, Hanser, München 1990.

U.S. 3,702,878 offenbart neue Organophosphor-Verbindungen und Verfahren zu deren Herstellung.

Die bekannten Stabilisatoren genügen nicht in jeder Hinsicht den hohen Anforderungen, die ein Stabilisator erfüllen soll, insbesondere hinsichtlich Lagerstabilität, Wasseraufnahme, Hydrolyseempfindlichkeit, Verarbeitungsstabilisierung, Farbverhalten, Flüchtigkeit, Migrationsverhalten, Verträglichkeit und Lichtschutzverbesserung. Es besteht deshalb weiterhin ein Bedarf an wirksamen Stabilisatoren für organische Materialien, die gegen oxidativen, thermischen und/oder lichtinduzierten Abbau empfindlich sind.

Es wurde nun gefunden, dass mittels eines neuen chemischen Verfahrens neue cyclische Phosphinsäurederivate herstellbar sind, die sich besonders gut als Stabilisatoren für organische Materialien, die gegen oxidativen, thermischen oder lichtinduzierten Abbau empfindlich sind, eignen. Insbesondere hervorzuheben ist die Eignung der genannten Verbindungen als Verarbeitungsstabilisatoren für synthetische Polymere.

Die vorliegende Erfindung betrifft daher Verbindungen der Formel I

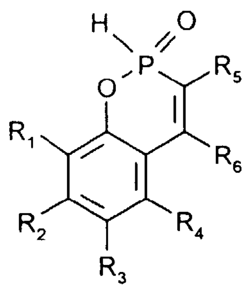

(I)

worin

$R_1$, $R_2$, $R_3$ und $R_4$ unabhängig voneinander Wasserstoff, Halogen, Hydroxy, $C_1$-$C_{25}$-Alkyl, durch Sauerstoff, Schwefel oder $\rangle$N- $R_7$ unterbrochenes $C_2$-$C_{25}$-Alkyl; $C_2$-$C_{24}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl; unsubstituiertes oder durch $C_1$-$C_4$-Alkyl substituiertes $C_5$-$C_{12}$-Cycloalkenyl; $C_1$-$C_{18}$-Alkoxy, $C_2$-$C_{24}$-Alkenyloxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoyl-amino, $C_3$-$C_{25}$-Alkenoyloxy, durch Sauerstoff, Schwefel oder $\rangle$N-$R_7$ unterbrochenes $C_3$-$C_{25}$-Alkanoyloxy; $C_5$-$C_9$-Cycloalkylcarbonyloxy, Benzoyloxy oder durch $C_1$-$C_{12}$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich -$(CH_2)_m$-$COR_8$ oder -$(CH_2)_nOR_9$ darstellt, oder wenn $R_2$ und $R_4$ Wasserstoff sind, $R_3$ zusätzlich einen Rest der Formel IIa, IIb oder IIc

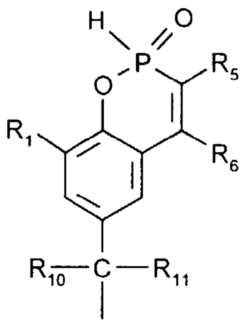

(IIa)

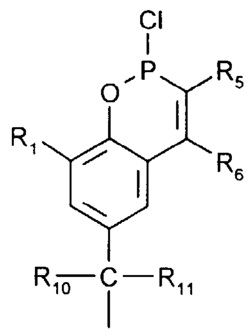

(IIb)

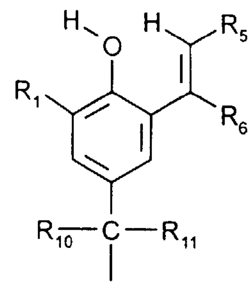

(IIc)

bedeutet,

$R_5$ und $R_6$ unabhängig voneinander Wasserstoff, $C_1$-$C_{25}$-Alkyl, durch Sauerstoff, Schwefel oder $>N-R_7$ unterbrochenes $C_2$-$C_{25}$-Alkyl; $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl darstellen, oder $R_5$ und $R_6$ zusammen mit den Kohlenstoffatomen, an die sie gebunden sind, ein unsubstituiertes $C_5$-$C_{12}$-Cycloalkenylen oder ein durch Sauerstoff, Schwefel oder $>N-R_7$ unterbrochenes unsubstituiertes $C_4$-$C_{11}$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert sind, $R_7$ Wasserstoff, $C_1$-$C_8$-Alkyl, $C_1$-$C_8$-Alkoxy, $C_1$-$C_8$-Alkanoyl oder unsubstituiertes oder mit $C_1$-$C_4$-Alkyl substituiertes Benzoyl bedeutet,

$R_8$ Hydroxy,

$\left[-O^{-}\ \frac{1}{r}M^{r+}\right]$ , $C_1$-$C_{18}$-Alkoxy oder 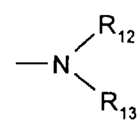

darstellt,

$R_9$ Wasserstoff, $C_1$-$C_8$-Alkanoyl oder unsubstituiertes oder mit $C_1$-$C_4$-Alkyl substituiertes Benzoyl bedeutet,

$R_{10}$ und $R_{11}$ unabhängig voneinander Wasserstoff, $CF_3$, $C_1$-$C_{12}$-Alkyl, unsubstituiertes oder durch $C_1$-$C_4$-Alkyl substituiertes Phenyl; oder-$(CH_2)_s$-$COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_{12}$-Cycloalkylidenring bilden,

$R_{12}$ und $R_{13}$ unabhängig voneinander Wasserstoff oder $C_1$-$C_{18}$-Alkyl darstellen,

$R_{14}$ Hydroxy, $C_1$-$C_{18}$-Alkoxy oder

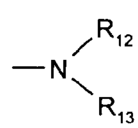

darstellt,

M ein r-wertiges Metallkation ist,

m 0, 1 oder 2 darstellt,

n 1, 2, 3, 4, 5 oder 6 bedeutet,

r 1, 2 oder 3 ist, und

s 0 oder 1 darstellt.

Die Verbindungen der Formel I können auch in einer tautomeren Form, wie in der Formel Ia dargestellt, vorliegen.

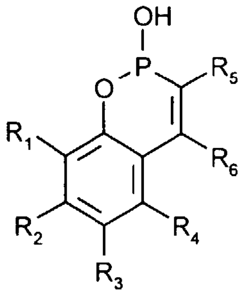

(Ia)

Es ist selbstverständlich, dass in der vorliegenden Erfindung von als Verbindungen der Formel 1 dargestellten Strukturen auch die tautomere Struktur der Formel Ia mitumfasst ist.

Halogen bedeutet beispielsweise Chlor, Brom oder Iod. Bevorzugt ist Chlor.

Alkyl mit bis zu 25 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Eine der bevorzugten Bedeutungen von $R_1$ $R_2$ $R_3$ und $R_4$ ist beispielweise $C_1$-$C_{18}$-Alkyl, insbesondere $C_1$-$C_{12}$-Alkyl, z.B. $C_1$-$C_{10}$-Alkyl. Eine besonders bevorzugte Bedeutung von $R_1$, $R_2$, $R_3$ und $R_4$ ist $C_1$-$C_8$-Alkyl, insbesondere $C_1$-$C_6$-Alkyl, z.B. $C_1$-$C_4$-Alkyl. Eine bevorzugte Bedeutung von $R_5$ und $R_6$ ist $C_1$-$C_{18}$-Alkyl, insbesondere $C_1$-$C_{12}$-Alkyl, z.B. $C_1$-$C_{10}$-Alkyl. Eine besonders bevorzugte Bedeutung von $R_5$ und $R_6$ ist $C_1$-$C_8$-Alkyl, insbesodere $C_1$-$C_6$-Alkyl, z.B. $C_1$-$C_4$-Alkyl wie beispielsweise Methyl. Eine bevorzugte Bedeutung von $R_7$ ist $C_1$-$C_6$-Alkyl, insbesondere $C_1$-$C_4$-Alkyl, z.B. Methyl, Ethyl oder n-Propyl. Eine bevorzugte Bedeutung von $R_{10}$ und $R_{11}$ ist $C_1$-$C_{10}$-Alkyl, insbesondere $C_1$-$C_8$-Alkyl, z.B. $C_1$-$C_6$-Alkyl. Eine besonders bevorzugte Bedeutung von $R_{10}$ und $R_{11}$ ist $C_1$-$C_4$-Alkyl, insbesondere $C_1$-$C_3$-Alkyl, z.B. Methyl. Eine bevorzugte Bedeutung von $R_{12}$ und $R_{13}$ ist $C_1$-$C_{12}$-Alkyl, insbesondere $C_1$-$C_{10}$-Alkyl, z.B. $C_1$-$C_8$-Alkyl. Eine besonders bevorzugte Bedeutung von $R_{12}$ und $R_{13}$ ist $C_1$-$C_6$-Alkyl, insbesondere $C_1$-$C_4$-Alkyl, z.B. Methyl oder Ethyl.

Durch Sauerstoff, Schwefel oder $>N-R_7$ unterbrochenes $C_2$-$C_{25}$-Alkyl bedeutet einen verzweigten oder unverzweigten Rest wie beispielsweise $CH_3$-O-$CH_2$-, $CH_3$-S-$CH_2$-, $CH_3$-NH-$CH_2$-, $CH_3$-N($CH_3$)-$CH_2$-, $CH_3$-O-$CH_2CH_2$-O-$CH_2$-, $CH_3$-(O-$CH_2CH_2$-$)_2$O-$CH_2$-, $CH_3$-(O-$CH_2CH_2$-$)_3$O-$CH_2$- oder $CH_3$-(O-$CH_2CH_2$-$)_4$O-$CH_2$-.

Alkenyl mit 3 bis 24 Kohlenstoffatomen bedeutet einen verzweigten oder unverzweigten Rest wie beispielsweise Vinyl, Propenyl, 2-Butenyl, 3-Butenyl, Isobutenyl, n-2,4-Pentadienyl, 3-Methyl-2-butenyl, n-2-Octenyl, n-2-Dodecenyl, iso-Dodecenyl, Oleyl, n-2-Octadecenyl oder n-4-Octadecenyl. Bevorzugt ist Alkenyl mit 2 bis 18, insbesondere 2 bis 12, z.B. 2 bis 6, vor allem 2 bis 4 Kohlenstoffatomen.

$C_7$-$C_9$-Phenylalkyl bedeutet beispielsweise Benzyl, α-Methylbenzyl, α,α-Dimethylbenzyl oder 2-Phenylethyl. Benzyl und α,α-Dimethylbenzyl ist bevorzugt.

Durch $C_1$-$C_4$-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-Butylphenyl, 2-Ethylphenyl oder 2,6-Diethylphenyl.

Unsubstituiertes oder durch $C_1$-$C_4$-Alkyl substituiertes $C_5$-$C_{12}$-Cycloalkyl bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl, Cyclooctyl, Cyclononyl, Cyclodecyl, Cycloundecyl oder Cyclododecyl. Bevorzugt ist unsubstituiertes oder durch $C_1$-$C_4$-Alkyl substituiertes $C_5$-$C_8$-Cycloalkyl, insbesondere $C_5$-$C_7$-Cycloalkyl, z.B. Cyclohexyl und tert-Butylcyclohexyl.

Unsubstituiertes oder durch $C_1$-$C_4$-Alkyl substituiertes $C_5$-$C_{12}$-Cycloalkenyl bedeutet beispielsweise Cyclopentenyl, Methylcyclopentenyl, Dimethylcyclopentenyl, Cyclohexenyl, Methylcyclohexenyl, Dimethylcyclohexenyl, Trimethylcyclohexenyl, tert-Butylcyclohexenyl, Cycloheptenyl, Cyclooctenyl, Cyclononenyl, Cyclodecenyl, Cycloundecenyl oder Cyclododecenyl. Bevorzugt ist unsubstituiertes oder durch $C_1$-$C_4$-Alkyl substituiertes $C_5$-$C_8$-Cycloalkenyl, insbesondere $C_5$-$C_7$-Cycloalkenyl, z.B. Cyclohexen-1-yl und tert-Butylcyclohexen-1-yl.

Alkoxy mit bis zu 18 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Bevorzugt ist Alkoxy mit 1 bis 12, insbesondere 1 bis 8, z.B. 1 bis 6

Kohlenstoffatomen. Eine besonders bevorzugte Bedeutung von $R_1$, $R_2$, $R_3$, $R_4$, $R_7$, $R_8$ und $R_{14}$ ist $C_1$-$C_4$-Alkoxy, insbesondere $C_1$-$C_3$-Alkoxy, z.B. Methoxy oder Ethoxy.

Alkenyloxy mit 2 bis 25 Kohlenstoffatomen bedeutet einen verzweigten oder unverzweigten Rest wie beispielsweise Vinyloxy, Propenyloxy, 2-Butenyloxy, 3-Butenyloxy, Isobutenyloxy, n-2,4-Pentadienyloxy, 3-Methyl-2-butenyloxy, n-2-Octenyloxy, n-2-Dodecenyloxy, iso-Dodecenyloxy, Oleyloxy, n-2-Octadecenyloxy oder n-4-Octadecenyloxy. Bevorzugt ist Alkenyloxy mit 3 bis 18, insbesondere 3 bis 12, z.B. 3 bis 6, vor allem 3 bis 4 Kohlenstoffatomen.

Alkylthio mit bis zu 18 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio, Heptylthio, Octylthio, Decylthio, Tetradecylthio, Hexadecylthio oder Octadecylthio. Bevorzugt ist Alkylthio mit 1 bis 12, insbesondere 1 bis 8, z.B. 1 bis 6 Kohlenstoffatomen.

Alkylamino mit bis zu 4 Kohlenstoffatomen bedeutet einen verzweigten oder unverzweigten Rest wie beispielsweise Methylamino, Ethylamino, Propylamino, Isopropylamino, n-Butyl-amino, Isobutylamino oder tert-Butylamino.

Di-($C_1$-$C_4$-alkyl)amino bedeutet auch, dass die beiden Reste unabhängig voneinander verzweigt oder unverzweigt sind wie beispielsweise Dimethylamino, Methylethylamino, Diethyl-amino, Methyl-n-propylamino, Methylisopropylamino, Methyl-n-butylamino, Methylisobutyl-amino, Ethylisopropylamino, Ethyl-n-butylamino, Ethylisobutylamino, Ethyl-tert-butylamino, Diethylamino, Diisopropylamino, Isopropyl-n-butylamino, Isopropylisobutylamino, Di-n-butyl-amino oder Di-isobutylamino.

Alkanoyloxy mit bis zu 25 Kohlenstoffatomen bedeutet einen verzweigten oder unverzweigten Rest wie beispielsweise Formyloxy, Acetoxy, Propionyloxy, Butanoyloxy, Pentanoyloxy, Hexanoyloxy, Heptanoyloxy, Octanoyloxy, Nonanoyloxy, Decanoyloxy, Undecanoyloxy, Dodecanoyloxy, Tridecanoyloxy, Tetradecanoyloxy, Pentadecanoyloxy, Hexadecanoyloxy, Heptadecanoyloxy, Octadecanoyloxy, Eicosanoyloxy oder Docosanoyloxy. Bevorzugt ist Alkanoyloxy mit 2 bis 18, insbesondere 2 bis 12, z.B. 2 bis 6 Kohlenstoffatomen. Besonders bevorzugt ist Acetoxy.

Alkanoylamino mit bis zu 25 Kohlenstoffatomen bedeutet einen verzweigten oder unverzweigten Rest wie beispielsweise Formylamino, Acetylamino, Propionylamino, Butanoyl-amino, Pentanoylamino, Hexanoylamino, Heptanoylamino, Octanoylamino, Nonanoylamino, Decanoylamino, Undecanoylamino, Dodecanoylamino, Tridecanoylamino, Tetradecanoyl-amino, Pentadecanoylamino, Hexadecanoylamino, Heptadecanoylamino, Octadecanoyl-amino, Eicosanoylamino oder Docosanoylamino. Bevorzugt ist Alkanoylamino mit 2 bis 18, insbesondere 2 bis 12, z.B. 2 bis 6 Kohlenstoffatomen.

Alkenoyloxy mit 3 bis 25 Kohlenstoffatomen bedeutet einen verzweigten oder unverzweigten Rest wie beispielsweise Propenyloxy, 2-Butenoyloxy, 3-Butenoyloxy, Isobutenoyloxy, n-2,4-Pentadienoyloxy, 3-Methyl-2-butenoyloxy, n-2-Octenoyloxy, n-2-Dodecenoyloxy, iso-Dodecenoyloxy, Oleoyloxy, n-2-Octadecenoyloxy oder n-4-Octadecenoyloxy. Bevorzugt ist Alkenoyloxy mit 3 bis 18, insbesondere 3 bis 12, z.B. 3 bis 6, vor allem 3 bis 4 Kohlenstoffatomen.

Durch Sauerstoff, Schwefel oder $>$N-$R_7$ unterbrochenes $C_3$-$C_{25}$-Alkanoyloxy bedeutet beispielsweise $CH_3$-O-$CH_2COO$-, $CH_3$-S-$CH_2COO$-, $CH_3$-NH-$CH_2COO$-, $CH_3$-N($CH_3$)-$CH_2COO$-, $CH_3$-O-$CH_2CH_2$-O-$CH_2COO$-, $CH_3$-(O-$CH_2CH_2$-$)_2$O-$CH_2COO$-, $CH_3$-(O-$CH_2CH_2$-$)_3$O-$CH_2COO$- oder $CH_3$-(O-$CH_2CH_2$-$)_4$O-$CH_2COO$-.

$C_5$-$C_9$-Cycloalkylcarbonyloxy bedeutet beispielsweise Cyclopentylcarbonyloxy, Cyclohexylcarbonyloxy, Cycloheptylcarbonyloxy, Cyclooctylcarbonyloxy oder Cyclononylcarbonyloxy. Cyclohexylcarbonyloxy ist bevorzugt.

Durch $C_1$-$C_{12}$-Alkyl substituiertes Benzoyloxy, das vorzugsweise 1 bis 3, insbesondere 1 oder 2 Alkylgruppen trägt, bedeutet beispielsweise o-, m- oder p-Methylbenzoyloxy, 2,3-Dimethyl-benzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy. Bevorzugte Substituenten sind $C_1$-$C_8$-Alkyl, insbesondere $C_1$-$C_4$-Alkyl.

Bilden $R_5$ und $R_6$ zusammen mit den Kohlenstoffatomen, an die sie gebunden sind, ein unsubstituiertes $C_5$-$C_{12}$-Cycloalkenylen, deren Ring gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert ist, bedeutet dies beispielsweise Cyclopentenylen, Cyclohexenylen, Cycloheptenylen, Cyclooctenylen, Cyclononenylen, Cyclodecenylen, Cycloundecenylen, Cyclododecenylen, Methylcyclopentenylen, Methylyclohexenylen, Methylcycloheptenylen, Methylcyclooctenylen, Methylcyclononenylen, Methylcyclodecenylen, Methylcycloundecenylen, Methylcyclododecenylen, Trimethylcyclopentenylen, Trimethylyclohexenylen, Trimethylcycloheptenylen, Trimethylcyclooctenylen, Trimethylcyclononenylen, Trimethylcyclodecenylen, Trimethylcycloundecenylen, Trimethylcyclododecenylen, Cyclohexylcyclopentenylen, Cyclohexylcyclohexenylen, Cyclohexylcycloheptenylen, Cyclohexylcyclooctenylen, Cyclohexylcyclononenylen, Cyclohexylcyclodecenylen, Cyclohexylcycloundecenylen, Cyclohexylcyclododecenylen, Phenylcyclopentenylen, Phenylcyclohexenylen, Phenylcycloheptenylen, Phenylcyclooctenylen, Phenylcyclononenylen, Phenylcyclodecenylen, Phenylcycloundecenylen, Phenylcyclododecenylen,

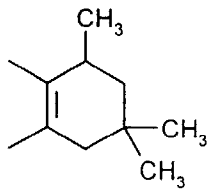 , 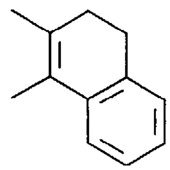 ,

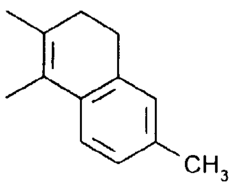 , 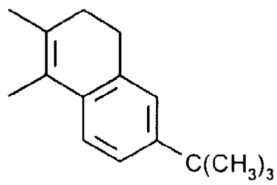 , 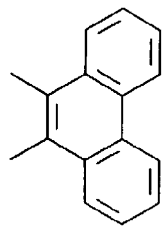 , 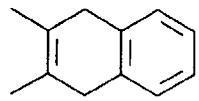 ,

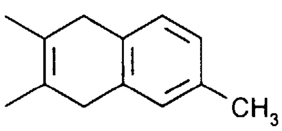 , 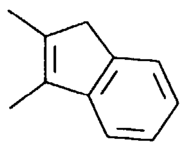 , 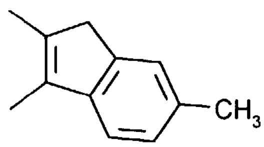 , 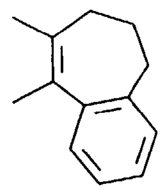 ,

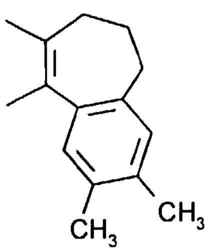 , 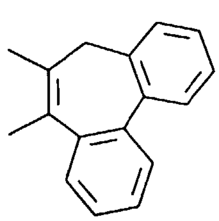 oder 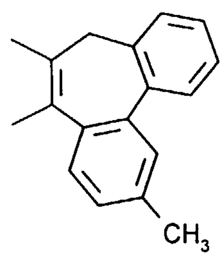 .

Bevorzugt bilden $R_5$ und $R_6$ zusammen mit den Kohlenstoffatomen, an die sie gebunden sind, ein unsubstituiertes $C_5$-$C_9$-Cycloalkenylen, insbesondere $C_5$-$C_8$-Cycloalkenylen, z.B. $C_5$-$C_7$-Cycloalkenylen, deren Ring gegebenenfalls mit $C_1$-$C_4$-Alkyl, unsubstituiertem oder durch $C_1$-$C_4$-Alkyl substituiertem $C_5$-$C_7$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert ist.

Bilden $R_5$ und $R_6$ zusammen mit den Kohlenstoffatomen, an die sie gebunden sind, ein durch Sauerstoff, Schwefel oder $>N-R_7$ unterbrochenes unsubstituiertes $C_4$-$C_{11}$-Cycloalkenylen, deren Ring gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert ist, bedeutet dies beispielsweise

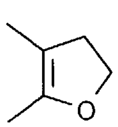 , 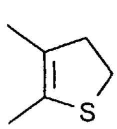 , 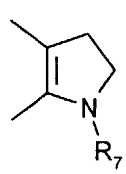 , 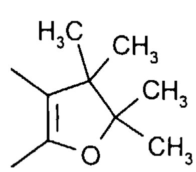 ,

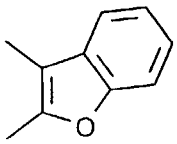 , 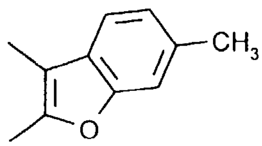 , 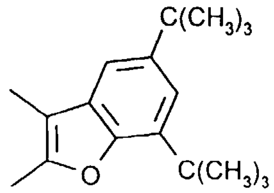 , 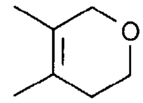 ,

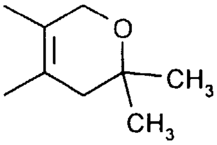 , 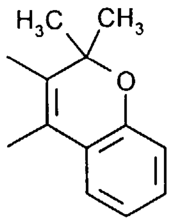 , 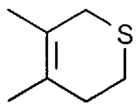 , 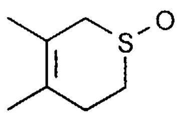 , 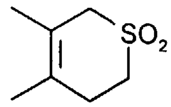 ,

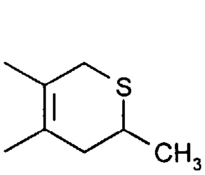 , 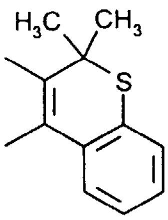 , 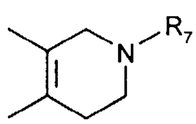 , 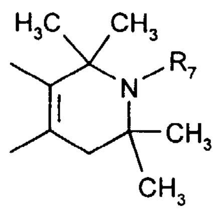

oder

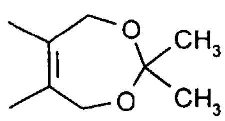 .

Bevorzugt bilden $R_5$ und $R_6$ zusammen mit den Kohlenstoffatomen, an die sie gebunden sind, ein durch Sauerstoff, Schwefel oder $\rangle$N-$R_7$ unterbrochenes unsubstituiertes $C_4$-$C_8$-Cycloalkenylen, insbesondere $C_5$-$C_8$-Cycloalkenylen, z. B. $C_5$-$C_7$-Cyclo-alkenylen, deren Ring gegebenenfalls mit $C_1$-$C_4$-Alkyl, unsubstituiertem oder durch $C_1$-$C_4$-Alkyl substituiertem Cyclohexyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert ist.

Alkanoyl mit bis zu 8 Kohlenstoffatomen bedeutet einen verzweigten oder unverzweigten Rest wie beispielsweise Formyl, Acetyl, Propionyl, Butanoyl, Pentanoyl, Hexanoyl, Heptanoyl oder Octanoyl. Bevorzugt ist Alkanoyl mit 1 bis 6, insbesondere 1 bis 4, z.B. 1 bis 3 Kohlenstoffatomen. Besonders bevorzugt ist Acetyl.

Durch $C_1$-$C_{12}$-Alkyl substituiertes Benzoyl, das vorzugsweise 1 bis 3, insbesondere 1 oder 2 Alkylgruppen trägt, bedeutet beispielsweise o-, m- oder p-Methylbenzoyl, 2,3-Dimethylbenzoyl, 2,4-Dimethylbenzoyl, 2,5-Dimethylbenzoyl, 2,6-Dimethylbenzoyl, 3,4-Dimethylbenzoyl, 3,5-Dimethylbenzoyl, 2-Methyl-6-ethylbenzoyl, 4-tert-Butylbenzoyl, 2-Ethylbenzoyl, 2,4,6-Trimethylbenzoyl, 2,6-Dimethyl-4-tert-butylbenzoyl oder 3,5-Di-tert-butylbenzoyl. Bevorzugte Substituenten sind $C_1$-$C_8$-Alkyl, insbesondere $C_1$-$C_4$-Alkyl.

$C_5$-$C_{12}$-Cycloalkenylen bedeutet eine ungesättigte Kohlenwasserstoffgruppe mit einer Doppelbindung, mit zwei freien Valenzen an der Doppelbindung und mindestens einer Ringeinheit und ist beispielsweise Cyclopentenylen, Cyclohexenylen, Cycloheptenylen, Cyclooctenylen, Cyclononenylen, Cyclodecenylen, Cycloundecenylen oder Cyclododecenylen.

Ein unsubstituierter oder durch $C_1$-$C_4$-Alkyl substituierter $C_5$-$C_{12}$-Cycloalkylidenring, der vorzugsweise 1 bis 3, insbesondere 1 bis 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist ein unsubstituierter oder durch $C_1$-$C_4$-Alkyl substituierter $C_5$-$C_9$-Cycloalkylidenring, insbesondere $C_5$-$C_8$-Cycloalkylidenring, z.

B. $C_5$-$C_7$-Cycloalkylidenring. Besonders bevorzugt ist Cyclohexyliden und tert-Butylcyclohexyliden.

Ein ein-, zwei- oder drei-wertiges Metallkation ist vorzugsweise ein Alkalimetall-, Erdalkalimetall- oder Aluminium-Kation, beispielsweise $Na^+$, $K^+$, $Mg^{++}$, $Ca^{++}$ oder $Al^{+++}$.

Bevorzugt sind die Verbindungen der Formel I, worin $R_2$ und $R_4$ Wasserstoff darstellen.

Bevorzugt sind auch die Verbindungen der Formel I, worin
$R_5$ und $R_6$ unabhängig voneinander Wasserstoff, $C_1$-$C_4$-Alkyl oder Phenyl darstellen, oder $R_5$ und $R_6$ zusammen mit den Kohlenstoffatomen, an die sie gebunden sind, ein unsubstituiertes $C_5$-$C_7$-Cycloalkenylen oder ein durch Sauerstoff oder Schwefel unterbrochenes unsubstituiertes $C_5$-$C_6$-Cycloalkenylen bilden, deren Ringe gegebenenfalls mit $C_1$-$C_4$-Alkyl oder Phenyl substituiert sind.

Ebenfalls bevorzugt sind die Verbindungen der Formel I, worin

$R_1$, $R_2$, $R_3$ und $R_4$ unabhängig voneinander Wasserstoff, Chlor, Hydroxy, $C_1$-$C_{18}$-Alkyl, durch Sauerstoff, Schwefel oder $>N-R_7$ unterbrochenes $C_2$-$C_{18}$-Alkyl; $C_2$-$C_{18}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes $C_5$-$C_8$-Cycloalkenyl; $C_1$-$C_{18}$-Alkoxy, $C_2$-$C_{18}$-Alkenyloxy, $C_1$-$C_{18}$-Alkylthio, $C_1$-$C_4$-Alkylamino, Di-($C_1$-$C_4$-alkyl)amino, $C_1$-$C_{18}$-Alkanoyloxy, $C_1$-$C_{18}$-Alkanoylamino, $C_3$-$C_{18}$-Alkenoyloxy, durch Sauerstoff, Schwefel oder $>N-R_7$ unterbrochenes $C_3$-$C_{18}$-Alkanoyloxy; $C_5$-$C_9$-Cycloalkylcarbonyloxy, Benzoyloxy oder durch $C_1$-$C_4$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich $-(CH_2)_m-COR_8$ oder $-(CH_2)_nOR_9$ darstellt, oder wenn $R_2$ und $R_4$ Wasserstoff sind, $R_3$ zusätzlich einen Rest der Formel IIa

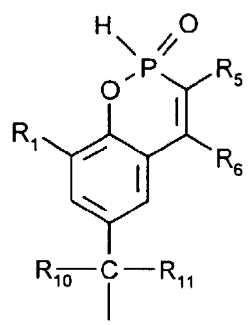

(IIa)

bedeutet,

$R_5$ und $R_6$ unabhängig voneinander Wasserstoff, $C_1$-$C_{18}$-Alkyl, durch Sauerstoff, Schwefel oder $>N-R_7$ unterbrochenes $C_2$-$C_{18}$-Alkyl; $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_8$-Cycloalkyl darstellen, oder $R_5$ und $R_6$ zusammen mit den Kohlenstoffatomen, an die sie gebunden sind, ein unsubstituiertes $C_5$-$C_9$-Cycloalkenylen oder ein durch Sauerstoff, Schwefel oder $>N-R_7$ unterbrochenes unsubstituiertes $C_4$-$C_8$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_8$-Cycloalkyl; unsubstituiertem oder mit $C_1$-$C_4$-Alkyl substituiertem Phenyl; oder mit Phenylen substituiert sind,

$R_7$ Wasserstoff, $C_1$-$C_6$-Alkyl, $C_1$-$C_6$-Alkoxy, $C_1$-$C_6$-Alkanoyl oder Benzoyl bedeutet,

$R_8$ Hydroxy,

$\left[-O^{-}\ \frac{1}{r}M^{r+}\right]$ , $C_1$-$C_{12}$-Alkoxy oder 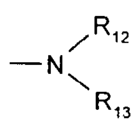

darstellt,

$R_9$ Wasserstoff, $C_1$-$C_8$-Alkanoyl oder Benzoyl bedeutet,

$R_{10}$ und $R_{11}$ unabhängig voneinander Wasserstoff, $CF_3$, $C_1$-$C_{10}$-Alkyl, unsubstituiertes oder durch $C_1$-$C_4$-Alkyl substituiertes Phenyl; oder $-(CH_2)_s-COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_9$-Cycloalkyliden-

ring bilden,
$R_{12}$ und $R_{13}$ unabhängig voneinander Wasserstoff oder $C_1$-$C_{12}$-Alkyl darstellen,
$R_{14}$ Hydroxy, $C_1$-$C_{12}$-Alkoxy oder

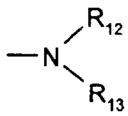

darstellt,
M ein Natrium, Kalium, Calcium oder Aluminium ist,
m 1 oder 2 darstellt,
n 2, 3, 4, 5 oder 6 bedeutet,
r1, 2 oder 3 ist, und
s 0 oder 1 darstellt.

Besonders bevorzugt sind die Verbindungen der Formel I, worin

$R_1$, $R_2$, $R_3$ und $R_4$ unabhängig voneinander Wasserstoff, Chlor, $C_1$-$C_{12}$-Alkyl, durch Sauerstoff oder Schwefel unterbrochenes $C_2$-$C_{12}$-Alkyl; $C_2$-$C_{12}$-Alkenyl, $C_7$-$C_9$-Phenylalkyl, Phenyl, $C_5$-$C_8$-Cycloalkyl, $C_5$-$C_8$-Cycloalkenyl, $C_1$-$C_{12}$-Alkoxy, $C_3$-$C_{12}$-Alkenyloxy, $C_1$-$C_{18}$-Alkylthio, $C_1$-$C_{12}$-Alkanoyloxy, $C_1$-$C_{12}$-Alkanoylamino, $C_3$-$C_{12}$-Alkenoyloxy, durch Sauerstoff oder Schwefel unterbrochenes $C_3$-$C_{12}$-Alkanoyloxy; $C_5$-$C_8$-Cycloalkylcarbonyloxy oder Benzoyloxy darstellen; $R_3$ zusätzlich einen Rest der Formel IIa

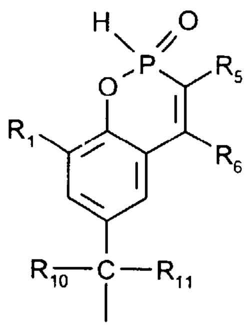

(IIa)

bedeutet,
$R_5$ und $R_6$ unabhängig voneinander Wasserstoff, $C_1$-$C_{12}$-Alkyl, durch Sauerstoff oder Schwefel unterbrochenes $C_2$-$C_{12}$-Alkyl; $C_7$-$C_9$-Phenylalkyl, Phenyl oder $C_5$-$C_8$-Cycloalkyl darstellen, oder $R_5$ und $R_6$ zusammen mit den Kohlenstoffatomen, an die sie gebunden sind, ein unsubstituiertes $C_5$-$C_9$-Cycloalkenylen oder ein durch Sauerstoff oder Schwefel unterbrochenes $C_4$-$C_8$-Cycloalkenylen bilden, deren Ringe gegebenenfalls mit $C_1$-$C_4$-Alkyl, $C_5$-$C_8$-Cycloalkyl oder Phenyl substituiert sind,
$R_{10}$ und $R_{11}$ unabhängig voneinander Wasserstoff, $CF_3$, $C_1$-$C_8$-Alkyl, Phenyl oder $-(CH_2)s-COR_{14}$ bedeuten, oder $R_{10}$ und $R_{11}$ zusammen mit dem Kohlenstoffatom, an das sie gebunden sind, einen $C_5$-$C_9$-Cycloalkylidenring bilden,
$R_{14}$ Hydroxy oder $C_1$-$C_{12}$-Alkoxy darstellt, und
s 0 oder 1 bedeutet.

Von besonderem Interesse sind die Verbindungen der Formel I, worin

$R_1$ Wasserstoff, $C_1$-$C_4$-Alkyl, Cyclohexyl, 1-Cyclohexenyl oder $C_7$-$C_9$-Phenylalkyl bedeutet,
$R_2$ Wasserstoff darstellt,
$R_3$ Wasserstoff, $C_1$-$C_4$-Alkyl, Cyclohexyl, 2-Cyclohexenyl, $C_7$-$C_9$-Phenylalkyl oder $C_1$-$C_4$-Alkoxy bedeutet,
$R_4$ Wasserstoff darstellt, und
$R_5$ und $R_6$ unabhängig voneinander Wasserstoff, $C_1$-$C_4$-Alkyl oder Phenyl darstellen, oder $R_5$ und $R_6$ zusammen

mit den Kohlenstoffatomen, an die sie gebunden sind, ein unsubstituiertes $C_5$-$C_7$-Cycloalkenylen oder ein durch Sauerstoff oder Schwefel unterbrochenes unsubstituiertes $C_5$-$C_6$-Cycloalkenylen bilden, deren Ringe gegebenenfalls mit $C_1$-$C_4$-Alkyl oder Phenyl substituiert sind.

Von ganz besonderem Interesse sind die Verbindungen der Formel I, worin

$R_1$ Wasserstoff, $C_1$-$C_4$-Alkyl oder $C_7$-$C_9$-Phenylalkyl bedeutet,
$R_2$ Wasserstoff darstellt,
$R_3$ $C_1$-$C_4$-Alkyl, $C_7$-$C_9$-Phenylalkyl oder $C_1$-$C_4$-Alkoxy bedeutet,
$R_4$ Wasserstoff darstellt, und
$R_5$ und $R_6$ unabhängig voneinander Wasserstoff oder Phenyl darstellen, oder $R_5$ und $R_6$ zusammen mit den Kohlenstoffatomen, an die sie gebunden sind, ein unsubstituiertes $C_5$-$C_7$-Cycloalkenylen oder ein durch Sauerstoff oder Schwefel unterbrochenes unsubstituiertes $C_5$-Cycloalkenylen bilden, deren Ringe gegebenenfalls mit $C_1$-$C_4$-Alkyl substituiert sind.

In der Literatur ist kein Verfahren bekannt, das die Herstellung der erfindungsgemässen Verbindungen der Formel I offenbart.

Ein weiterer Gegenstand der Erfindung ist deshab ein Verfahren zur Herstellung von Verbindungen der Formel I

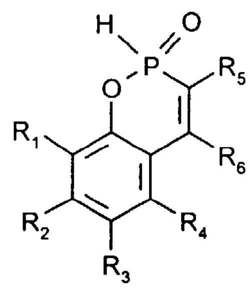

(I)

worin die allgemeinen Symbole die angegebenen Bedeutungen haben, dadurch gekennzeichnet, dass eine Verbindung der Formel III

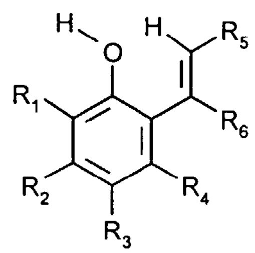

(III)

worin die allgemeinen Symbole die angegebenen Bedeutungen haben, mit Phosphortrichlorid zu einer Verbindung der Formel IV

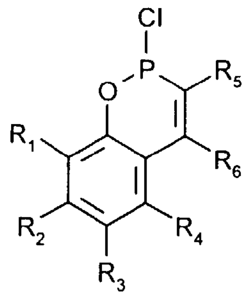

(IV)

worin die allgemeinen Symbole die angegebenen Bedeutungen haben, umgesetzt wird, und diese anschliessend ohne Isolierung mit Wasser hydrolisiert wird.

Die Verbindungen der Formel IV können isoliert und charakterisiert werden.

Die Umsetzung der Verbindung der Formel III mit Phosphortrichlorid erfolgt in der Schmelze oder in Gegenwart eines geeigneten organischen, polaren oder apolaren, aprotischen Lösungsmittels. Bevorzugt geschieht diese Umsetzung bei einer Temperatur zwischen -20°C und dem Siedepunkt des Lösungsmittels oder Phosphortrichlorids, insbesondere bei Temperaturen zwischen 20 und 200°C, z.B. 20 und 150°C. Besonders bevorzugt wird diese Umsetzung ohne Lösungsmittel durchgeführt. Das Phosphortrichlorid ist dabei sowohl Lösungsmittel wie auch Reaktionspartner. Das Phosphortrichlorid wird bevorzugt in einem molaren Überschuss bezüglich der Verbindung der Formel III verwendet. Von besonderem Interesse ist ein Molverhältnis der Verbindung der Formel III zu Phosphortrichlorid von 1 : 20 bis 1:1, insbesondere 1:10 bis 1 : 1, z.B. 1 : 5 bis 1:1.

Geeignete Lösungsmittel zur Durchführung der Reaktion sind u.a. Kohlenwasserstoffe (beispielsweise Mesitylen, Toluol, Hexan, Pentan oder weitere Petroletherfraktionen), halogenierte Kohlenwasserstoffe (beispielsweise Di- oder Trichlormethan, 1,2-Dichlorethan, 1,1,1 Trichlorethan oder Chlorbenzol), Ether (z.B. Diethylether, Dibutylether, Dioxan oder Tetrahydrofuran), Ketone (z.B. Aceton, Ethylmethylketon, Diethylketon, Methylpropylketon oder Cyclohexanon), ferner Acetonitril oder Butylacetat. Ein besonders bevorzugtes Lösungsmittel ist Toluol.

Bemerkenswert ist die Tatsache, dass die Umsetzung der Verbindungen der Formel III mit Phosphortrichlorid zu den Verbindungen der Formel IV ohne Katalysatoren wie beispielsweise Friedel-Crafts-Katalysatoren, insbesondere Aluminiumchlorid, in bereits relativ kurzen Reaktionszeiten erfolgt.

Die Umsetzung der Verbindungen der Formel III mit Phosphortrichlorid zu den Verbindungen der Formel IV kann auch in Gegenwart einer Base erfolgen.

Die Base kann in unterschiedlichen Mengen eingesetzt werden, von katalytischen über stöchiometrische Mengen bis hin zu mehrfachem molarem Überschuss bezüglich eingesetzten Verbindungen der Formel III. Der bei der Reaktion gebildete Halogenwasserstoff wird gegebenenfalls durch die Base in Halogenid überführt, das durch Filtration und/ oder Waschen mit einer geeigneten wässrigen oder festen Phase entfernt werden kann; dabei kann auch ein zweites, nicht mit Wasser mischbares Lösungsmittel eingesetzt werden. Die Isolierung der Produkte erfolgt zweckmäßig durch Eindampfen der organischen Phase und Trocknung des Rückstandes.

Geeignete Basen sind u.a. primäre, sekundäre oder vor allem tertiäre Amine (z.B. Trimethylamin, Triethylamin, Tributylamin, N,N-Dimethylanilin, N,N-Diethylanilin oder Pyridin), Alkoholate (z.B. Natriummethanolat) oder Alkalicarbonate (z.B. Natriumcarbonat oder Kaliumcarbonat). Besonders bevorzugt sind tertiäre Amine, insbesondere Triethylamin.

Die Hydrolyse der Verbindungen der Formel IV mit Wasser zu den Verbindungen der Formel I erfolgt bevorzugt bei einer Temperatur von -20 bis 150°C, insbesondere -10 bis 120°C, z.B. 0 bis 110°C. Dabei kann das Wasser bezüglich der Verbindung der Formel IV in einem grossen molaren Ueberschuss verwendet werden. In einer besonders bevorzugten Ausführungsform wird das heisse, rohe Reaktionsgemisch, das die Verbindungen der Formel IV enthält, ohne Aufarbeitung in einen grossen Ueberschuss Wasser gegossen. Die Verbindungen der Formel I kristallisieren dabei aus oder können mit einem geeigneten Lösungsmittel wie beispielsweise Essigsäureethylester, Diethylether oder Dichlormethan extrahiert werden.

Die Verbindungen der Formel IV sind in der Literatur unbekannt.

Die vorliegende Erfindung betrifft daher auch neue Verbindungen der Formel IV

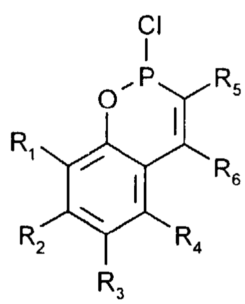

(IV)

worin

$R_1$, $R_2$, $R_3$ und $R_4$ unabhängig voneinander Wasserstoff, Halogen, Hydroxy, $C_1$-$C_{25}$-Alkyl, durch Sauerstoff, Schwefel oder >N- $R_7$ unterbrochenes $C_2$-$C_{25}$-Alkyl; $C_2$-$C_{24}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl;

unsubstituiertes oder durch $C_1$-$C_4$-Alkyl substituiertes $C_5$-$C_{12}$-Cycloalkenyl; $C_1$-$C_{18}$-Alkoxy, $C_2$-$C_{24}$-Alkenyloxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoyl-amino, $C_3$-$C_{25}$-Alkenoyloxy, durch Sauerstoff, Schwefel oder $>N-R_7$ unterbrochenes $C_3$-$C_{25}$-Alkanoyloxy; $C_5$-$C_9$-Cycloalkylcarbonyloxy, Benzoyloxy oder durch $C_1$-$C_{12}$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich $-(CH_2)_m-COR_8$ oder $-(CH_2)_nOR_9$ darstellt, oder wenn $R_2$ und $R_4$ Wasserstoff sind, $R_3$ zusätzlich einen Rest der Formel IIa, IIb oder IIc

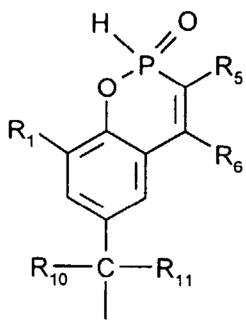
(IIa)

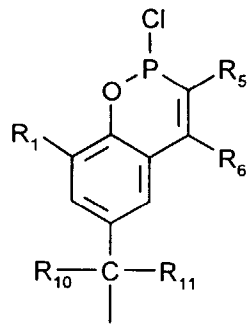
(IIb)

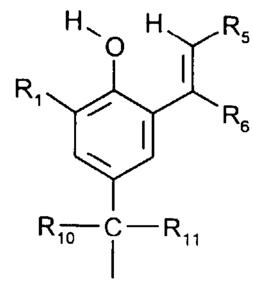
(IIc)

bedeutet,

$R_5$ und $R_6$ unabhängig voneinander Wasserstoff, $C_1$-$C_{25}$-Alkyl, durch Sauerstoff, Schwefel oder $>N-R_7$ unterbrochenes $C_2$-$C_{25}$-Alkyl; $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl darstellen, oder $R_5$ und $R_6$ zusammen mit den Kohlenstoffatomen, an die sie gebunden sind, ein unsubstituiertes $C_5$-$C_{12}$-Cycloalkenylen oder ein durch Sauerstoff, Schwefel oder $>N-R_7$ unterbrochenes unsubstituiertes $C_4$-$C_{11}$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert sind,

$R_7$ Wasserstoff, $C_1$-$C_8$-Alkyl, $C_1$-$C_8$-Alkoxy, $C_1$-$C_8$-Alkanoyl oder unsubstituiertes oder mit $C_1$-$C_4$-Alkyl substituiertes Benzoyl bedeutet,

$R_8$ Hydroxy,

$\left[-O^{-}\ \frac{1}{r}M^{r+}\right]$ , $C_1$-$C_{18}$-Alkoxy oder 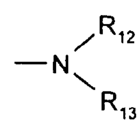

darstellt,

$R_9$ Wasserstoff, $C_1$-$C_8$-Alkanoyl oder unsubstituiertes oder mit $C_1$-$C_4$-Alkyl substituiertes Benzoyl bedeutet,

$R_{10}$ und $R_{11}$ unabhängig voneinander Wasserstoff, $CF_3$, $C_1$-$C_{12}$-Alkyl, unsubstituiertes oder durch $C_1$-$C_4$-Alkyl substituiertes Phenyl; oder $-(CH_2)_s-COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_{12}$-Cycloalkylidenring bilden,

$R_{12}$ und $R_{13}$ unabhängig voneinander Wasserstoff oder $C_1$-$C_{18}$-Alkyl darstellen,

$R_{14}$ Hydroxy, $C_1$-$C_{18}$-Alkoxy oder

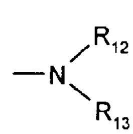

darstellt,
M ein r-wertiges Metallkation ist,
m 0, 1 oder 2 darstellt,
n 1, 2, 3, 4, 5 oder 6 bedeutet,
r 1, 2 oder 3 ist, und
s 0 oder 1 darstellt.

Bevorzugte Gruppen von neuen Verbindungen der Formel IV entsprechen den oben für die Verbindungen der Formel I ausgedrückten Bevorzugungen.

Die Verbindungen der Formel III sind in der Literatur teilweise bekannt und können in Analogie zu U.S. 5,414,033, Beispiel 1; oder EP-A-0 705 870 hergestellt werden.

Die vorliegende Erfindung betrifft daher auch neue Verbindungen der Formel III

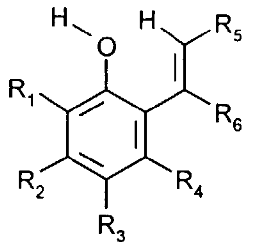

(III)

$R_1$, $R_2$, $R_3$ und $R_4$ unabhängig voneinander Wasserstoff, Halogen, Hydroxy, $C_1$-$C_{25}$-Alkyl, durch Sauerstoff, Schwefel oder $>$N-$R_7$ unterbrochenes $C_2$-$C_{25}$-Alkyl; $C_2$-$C_{24}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl; unsubstituiertes oder durch $C_1$-$C_4$-Alkyl substituiertes $C_5$-$C_{12}$-Cycloalkenyl; $C_1$-$C_{18}$-Alkoxy, $C_2$-$C_{24}$-Alkenyloxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoyl-amino, $C_3$-$C_{25}$-Alkenoyloxy, durch Sauerstoff, Schwefel oder $>$N-$R_7$ unterbrochenes $C_3$-$C_{25}$-Alkanoyloxy; $C_5$-$C_9$-Cycloalkylcarbonyloxy, Benzoyloxy oder durch $C_1$-$C_{12}$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich $-(CH_2)_m-COR_8$ oder $-(CH_2)_nOR_9$ darstellt, oder wenn $R_2$ und $R_4$ Wasserstoff sind, $R_3$ zusätzlich einen Rest der Formel IIa, IIb oder IIc

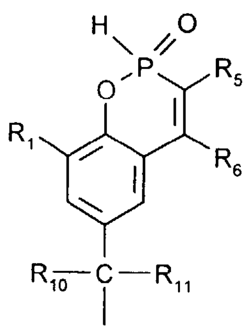

(IIa)

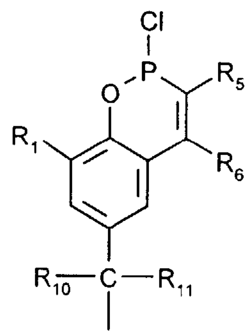

(IIb)

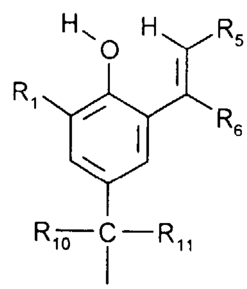

(IIc)

bedeutet,
$R_5$ und $R_6$ zusammen mit den Kohlenstoffatomen, an die sie gebunden sind, ein unsubstituiertes Cyclopentenylen oder $C_7$-$C_{12}$-Cycloalkenylen oder ein durch Sauerstoff, Schwefel oder $>$N-$R_7$ unterbrochenes unsubstituiertes $C_4$-$C_{11}$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert sind,
$R_7$ Wasserstoff, $C_1$-$C_8$-Alkyl, $C_1$-$C_8$-Alkoxy, $C_1$-$C_8$-Alkanoyl oder unsubstituiertes oder mit $C_1$-$C_4$-Alkyl substituier-

tes Benzoyl bedeutet,
$R_8$ Hydroxy,

$\left[-O^{-}\ \frac{1}{r}M^{r+}\right]$, $C_1$-$C_{18}$-Alkoxy oder 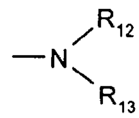

darstellt,
$R_9$ Wasserstoff, $C_1$-$C_8$-Alkanoyl oder unsubstituiertes oder mit $C_1$-$C_4$-Alkyl substituiertes Benzoyl bedeutet,
$R_{10}$ und $R_{11}$ unabhängig voneinander Wasserstoff, $CF_3$, $C_1$-$C_{12}$-Alkyl, unsubstituiertes oder durch $C_1$-$C_4$-Alkyl substituiertes Phenyl; oder $-(CH_2)_s-COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_{12}$-Cycloalkylidenring bilden,
$R_{12}$ und $R_{13}$ unabhängig voneinander Wasserstoff oder $C_1$-$C_{18}$-Alkyl darstellen,
$R_{14}$ Hydroxy, $C_1$-$C_{18}$-Alkoxy oder

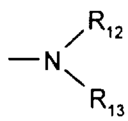

darstellt,
M ein r-wertiges Metallkation ist,
m 0, 1 oder 2 darstellt,
n 1, 2, 3, 4, 5 oder 6 bedeutet,
r 1, 2 oder 3 ist, und
s 0 oder 1 darstellt.

Bevorzugte Gruppen von neuen Verbindungen der Formel III entsprechen den oben für die Verbindungen der Formel I ausgedrückten Bevorzugungen.

Besonders bevorzugt sind die Verbindungen der Formel III, worin

$R_1$ Wasserstoff, $C_1$-$C_4$-Alkyl oder $C_7$-$C_9$-Phenylalkyl bedeutet,
$R_2$ Wasserstoff darstellt,
$R_3$ $C_1$-$C_4$-Alkyl, $C_7$-$C_9$-Phenylalkyl oder $C_1$-$C_4$-Alkoxy bedeutet,
$R_4$ Wasserstoff darstellt, und
$R_5$ und $R_6$ zusammen mit den Kohlenstoffatomen, an die sie gebunden sind, ein unsubstituiertes Cyclopentenylen oder Cycloheptenylen oder ein durch Sauerstoff oder Schwefel unterbrochenes unsubstituiertes $C_5$-Cycloalkenylen bilden, deren Ringe gegebenenfalls mit $C_1$-$C_4$-Alkyl substituiert sind.

Die Herstellung der neuen Verbindungen der Formel III erfolgt beispielsweise, und dies ist bevorzugt, durch Kondensation der mindestens in einer ortho-Position unsubstituierten Phenole der Formel V

(V) 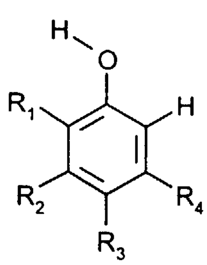

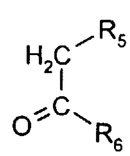 (VI)

mit einem Keton der Formel VI, worin die allgemeinen Symbole die angegebene Bedeutung haben. Die Reaktion wird

bei erhöhter Temperatur, insbesondere Temperaturen von 20 bis 100°C in der Schmelze oder in einem Lösungsmittel, gegebenenfalls unter leichtem Druck, durchgeführt. Bevorzugt wird die Reaktion in der Schmelze in einem Temperaturbereich von 20 bis 80°C, insbesondere 30 bis 60°C, durchgeführt. Die Reaktion kann durch Zusatz einer Säure wie Salzsäure, Schwefelsäure oder Methansulfonsäure katalysiert werden. Bevorzugt wird Salzsäure-Gas verwendet.

Die erfindungsgemässen Verbindungen der Formel I eignen sich zum Stabilisieren von organischen Materialien gegen oxidativen, thermischen oder lichtinduzierten Abbau.

Beispiele für derartige Materialien sind:

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), (VLDPE) und (ULDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo-und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide, Polyetherimide, Polyesterimide, Polyhydantoine und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.

26. Vernetzte Epoxidharze, die sich von aliphatischen, cycloaliphatischen, heterocyclischen oder aromatischen Glycidylverbindungen ableiten, z.B. Produkte von Bisphenol-A-diglycidyl-ethern, Bisphenol-F-diglycidylethern, die mittels üblichen Härtern wie z.B. Anhydriden oder Aminen mit oder ohne Beschleunigern vernetzt werden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/-EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/-Acrylat, POM/MBS, PPO/HIPS, PPO/PA 6.6 und Copolymere, PA/HDPE, PA/PP, PA/PPO, PBT/PC/ABS oder PBT/PET/PC.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher auch Zusammensetzungen enthaltend (a) ein dem oxidativen, thermischen oder lichtinduzierten Abbau unterworfenes organisches Material und (b) mindestens eine Verbindung der Formel I.

Vorzugsweise handelt es sich bei den zu schützenden organischen Materialien um natürliche, halbsynthetische oder bevorzugt synthetische Polymere. Besonders bevorzugt sind thermoplastische Polymere, insbesondere PVC oder Polyolefine, vor allem Polyethylen und Polypropylen. Von besonderem Interesse ist auch ein Copolymer oder Pfropfcopolymer von Styrol oder $\alpha$-Methylstyrol mit Dienen, Polybutadien oder Acrylderivaten. Ganz besonders bevorzugt ist eine ABS-Spritzguss.

Besonders hervorzuheben ist die Wirkung der erfindungsgemässen Verbindungen gegen thermischen und oxidativen Abbau, vor allem bei thermischer Belastung, wie sie bei der Verarbeitung von Thermoplasten auftritt. Die erfindungsgemässen Verbindungen sind daher hervorragend als Verarbeitungsstabilisatoren einzusetzen.

Vorzugsweise werden die Verbindungen der Formel I dem zu stabilisierenden Material in Mengen von 0,01 bis 10 %, beispielsweise 0,01 bis 5 %, vorzugsweise 0,025 bis 3 %, insbesondere 0,025 bis 1 % zugesetzt, bezogen auf das Gewicht des zu stabilisierenden organischen Materials.

Zusätzlich zu den Verbindungen der Formel I können die erfindungsgemässen Zusammensetzungen weitere Costabilisatoren (Additive) enthalten, wie beispielsweise die folgenden:

1. Antioxidantien

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Dinonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxy-anisol, 3,5-Di-tert-butyl-4-hydroxyphenyl-stearat, Bis(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.

1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonyl-phenol], 2,2'-Methylen-bis[6-(α,α-dimethylbenzyl)-4-nonylphenol], 4,4'-Methylen-bis(2, 6-ditert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1, 3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis [3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5, 5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzyl-ether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2, 6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.

1.9. Hydroxybenzyl-Aromaten, z. B. 1,3,5-Tris(3,5-di-tert-butyl-4-hydroxybenzyl)-2,4,6-trime-thylbenzol, 1,4-Bis (3,5-di-tert-butyl-4-hydroxybenzyl)-2,3,5,6-tetramethylbenzol, 2,4,6-Tris-(3, 5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3, 5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3, 5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxy-benzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N, N'-Bis(hydroxyethyl)-oxalsäurediamid, 3-Thiaundecanol, 3-Thiapentadecanol, Trimethylhexandiol, Trimethylolpropan, 4-Hydroxymethyl-1 -phospha-2,6,7-trioxabicyclo-[2.2.2]-octan.

1.17. Amide der β-(3,5-Di-tert-butyl-4-hydroxyphenyl)-propionsäure, wie z.B. N,N'-Bis(3,5-di-tert-butyl-4-hydroxyphenylpropionyl)-hexamethylendiamid, N,N'-Bis(3,5-di-tert-buty-4-hydroxyphenylpropionyl)-trimethylendiamid, N, N'-Bis(3,5-di-tert-butyl-4-hydroxyphenylpropionyl)-hydrazid, N,N'-Bis[2-(3-[3,5-di-tert-butyl-4-hydroxyphenyl]-propionyloxy)ethyl]oxamid (Naugard®XL-1 der Firma Uniroyal).

1.18. Ascorbinsäure (Vitamin C).

1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N, N'-Bis(1-methyl-heptyl)-p-phenylendiamin, N,N'-Dicyclohexyl-p-phenylendiamin, N,N'-Diphenyl-p-phenylendiamin, N,N'-Di-(2-naphthyl)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylaminophenol, 4-Nonanoylamino-phenol, 4-Dodecanoylamino-phenol, 4-Octadecanoylaminophenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethypiperidin4-on, 2,2,6,6-Tetramethylpiperidin-4-ol.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1 ,1 ,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxy-carbonylethyl)phenyl)-benzotriazol, 2-(3'-tert-Butyl-2'-hydroxy-5'-(2-octyloxycarbonylethyl)-phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, 2-(3'-tert-Butyl-2'-hydroxy-5'-(2-isooctyloxycarbonylethyl)phenyl-benzotriazol, 2,2'-Methylen-bis[4-(1 ,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300;

$$\left[R-CH_2CH_2-COO-CH_2CH_2\right]_2$$

mit R = 3'-tert-Butyl-4'-hydroxy-5'-2H-benzotriazol-2-yl-phenyl; 2-[2,-Hydroxy-3'-(α,α-dimethylbenzyl)-5'-(1,1,3,3-tetramethylbutyl)-phenyl]-benzotriazol; 2-[2'-Hydroxy-3'-(1,1,3,3-tetramethylbutyl)-5'-(α,α-dimethylbenzyl)-phenyl]-benzotriazol

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3, 5-Di-tert-butyl-4-hydroxybenzoesäure-2 ,4-di-tert-butylphenylester, 3,5-Di-tert-butyl-4-hydroxybenzoesäurehexadecylester, 3, 5-Di-tert-butyl-4-hydroxybenzoe-säure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäure-methylester, α-Cyano-β-methyl-p-methoxy-zimtsäuremethylester bzw. butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis[4-(1,1,3,3-tetramethyl-butyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkyl-estern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis(2,2,6,6-tetramethyl-piperidin-4-yl)-sebacat, Bis-(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, lineare oder cyclische Kondensationsprodukte aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis (2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3, 5-di-tert-butylbenzyl)-malonat, 3-n-Octyl-7,7,9,9-tetramethyl-1,3,8-triazaspiro [4.5]-decan-2,4-dion, Bis(1-octyloxy-2,2,6,6-tetramethylpiperidyl)-sebacat, Bis(1-octyloxy-2,2,6,6-tetramethylpiperidyl)-succinat, lineare oder cyclische Kondensationsprodukte aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondensationsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro-[4,5]decan und Epichlorhydrin, 1,1-Bis(1,2,2,6,6-pentamethyl-4-piperidyloxycarbonyl)-2-(4-methoxyphenyl)-ethen, N,N'-Bis-formyl-N,N'-bis(2,2,6,6-tetramethyl-4-piperidyl)-hexamethylendiamin, Diester der 4-Methoxy-methylen-malonsäure mit 1,2,2,6,6-Pentamethyl-4-hydroxy-piperidin, Poly[methylpropyl-3-oxy-4-(2,2,6,6-tetramethyl-4-piperidyl)]-siloxan, Reaktionsprodukt aus Maleinsäureanhydrid-α-olefin-copolymer und 2,2,6,6-Tetramethyl-4-aminopiperidin oder 1,2,2,6,6-Pentamethyl-4-aminopiperidin.

2.7. Oxalsäurediamide, wie z.B. 4,4'-Di-octyloxy-oxanilid, 2,2'-Diethoxy-oxanilid, 2,2'-Di-octyloxy-5, 5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o-und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1 ,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxy-propoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin, 2-{2-Hydroxy-4-[3-(2-ethylhexyl-1-oxy)-2-hydroxypropyloxy]phenyl}-4,6-bis(2,4-dimethylphenyl)-1,3,5-triazin.

3. Metalldesaktivatoren, wie z.B. N,N'-Diphenyloxalsäurediamid, N-Salicylal-N'-salicyloyl-hydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, Isophthalsäure-dihydrazid, Sebacinsäure-bis-phenylhydrazid, N, N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris (nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methyl-phenyl)-pentaerythritdiphosphit, Bis-isodecyloxy-pentaerythritdiphosphit, Bis (2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis(2,4-di-tert-butyl-6-methylphenyl)-methylphosphit, Bis(2,4-di-tert-butyl-6-methylphenyl)-ethylphosphit, 2,2',2''-Nitrilo[triethyl-tris(3,3',5,5'-tetra-tert-butyl-1,1'-biphenyl-2' ,2'-diyl)-phosphit], 2-Ethylhexyl-(3,3',5,5'-tetra-tert-butyl-1,1'-biphenyl-2,2'-diyl)-phosphit.

Besonders bevorzugt werden die folgenden Phosphite verwendet:
Tris(2,4-di-tert-butylphenyl)-phosphit (Irgafos®168, Ciba-Geigy), Tris(nonylphenyl)-phosphit,

(A) 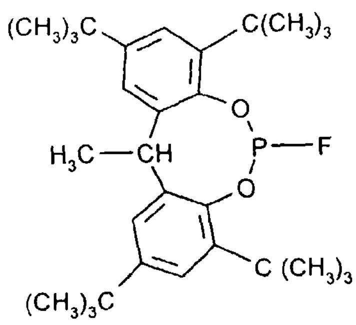

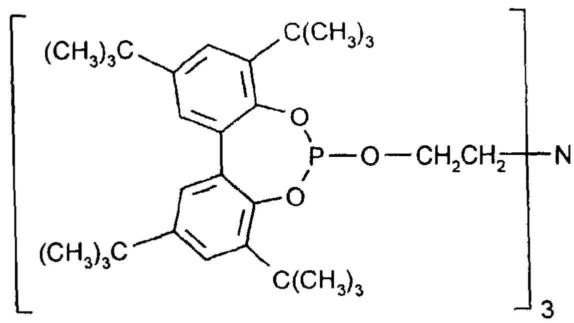 (B)

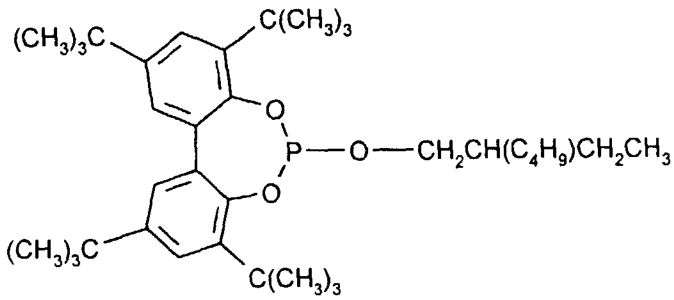 (C)

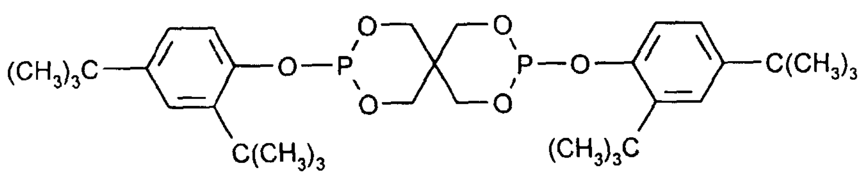 (D)

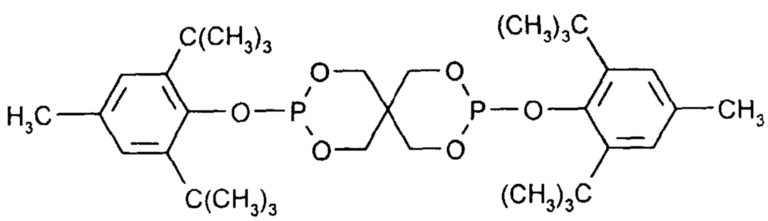 (E)

(F) 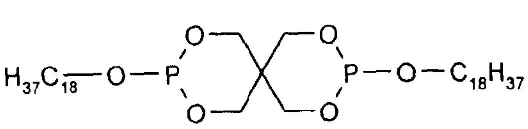

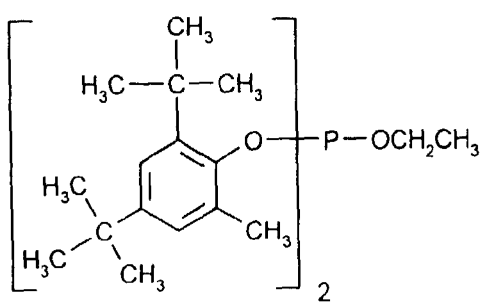 (G)

5. <u>Hydroxylamine</u> wie z.B. N,N-Dibenzylhydroxylamin, N,N-diethylhydroxylamin, N,N-Dioctyl-hydroxylamin, N,N-Dilaurylhydroxylamin, N,N-Ditetradecylhydroxylamin, N, N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.

6. Nitrone wie z.B. N-Benzyl-alpha-phenyl-nitron, N-Ethyl-alpha-methyl-nitron, N-Octyl-alphaheptyl-nitron, N-Lauryl-alpha-undecyl-nitron, N-Tetradecyl-alpha-tridecyl-nitron, N-Hexadecyl-alpha-pentadecyl-nitron, N-Octadecyl-alpha-heptadecyl-nitron, N-Hexadecyl-alphaheptadecyl-nitron, N-Octadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.

7. Thiosynergisten wie z.B. Thiodipropionsäure-di-laurylester oder Thiodipropionsäure-distearylester.

8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.

9. Polyamidstabilisatoren, wie z.B. Kupfersalze in Kombination mit Jodiden und/oder Phosphorverbindungen und Salze des zweiwertigen Mangans.

10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkaliund Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinkbrenzcatechinat.

11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere")

12. Füllstoffe und Verstärkungsmittel, wie z.B. Calciumcarbonat, Silikate, Glasfasern, Glaskugeln, Talk, Kaolin, Glimmer, Bariumsulfat, Metalloxide und -hydroxide, Ruß, Graphit, Holzmehl und Mehle oder Fasern anderer Naturprodukte, synthetische Fasern.

13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.

14. Benzofuranone bzw. Indolinone, wie z.B. in U.S. 4,325,863; U.S. 4,338,244; U.S.5,175,312, U.S. 5,216,052; U.S. 5,252,643; DE-A-4316611; DE-A-4316622; DE-A-4316876; EP-A-0589839 oder EP-A-0591102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxylphenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on, 3-(3,4-Dimethylphenyl)-5,7-di-tert-butyl-benzofuran-2-on, 3-(2,3-Dimethylphenyl)-5,7-di-tert-butyl-benzofuran-2-on.

Die Costabilisatoren, mit Ausnahme der unter Punkt 14 aufgeführten Benzofuranone, werden beispielsweise in Konzentrationen von 0,01 bis 10 %, bezogen auf das Gesamtgewicht des stabilisierenden organischen Materials, zugesetzt.

Weitere bevorzugte Zusammensetzungen enthalten neben der Komponente (a) und den Verbindungen der Formel I noch weitere Additive, insbesondere phenolische Antioxidantien, Lichtschutzmittel oder/und Verarbeitungsstabilisatoren.

Besonders bevorzugte Additive sind phenolische Antioxidantien (Punkt 1 der Liste), sterisch gehinderte Amine (Punkt 2.6 der Liste), Phosphite und Phosphonite (Punkt 4 der Liste) und peroxidzerstörende Verbindungen (Punkt 8 der Liste).

Ebenfalls besonders bevorzugte zusätzliche Additive (Stabilisatoren) sind Benzofuran-2-one, wie sie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 und EP-A-0 591 102 beschrieben werden.

Beispiele für solche Benzofuran-2-one sind Verbindungen der Formel

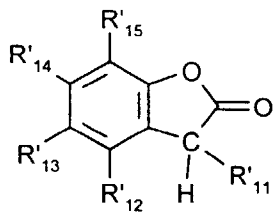

worin

$R'_{11}$ ein unsubstituiertes oder substituiertes carbocyclisches oder heterocyclisches aromatisches Ringsystem bedeutet;
$R'_{12}$ Wasserstoff ist;
$R'_{14}$ Wasserstoff, Alkyl mit 1 bis 12 Kohlenstoffatomen, Cyclopentyl, Cyclohexyl oder Chlor ist;
$R'_{13}$ die Bedeutung von $R'_{12}$ oder $R'_{14}$ hat oder ein Rest der Formel

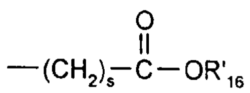 ,

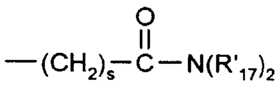 , 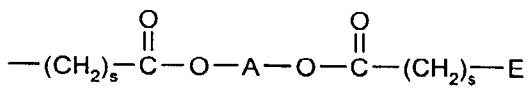 ,

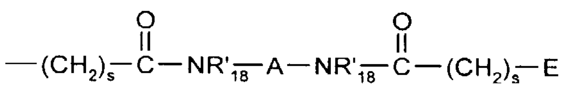 , 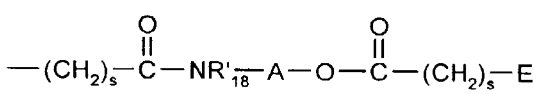 ,

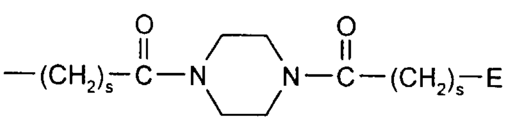 , $-CH_2-S-R'_{19}$, 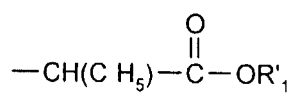

oder -D-E ist, worin
$R'_{16}$ Wasserstoff, Alkyl mit 1 bis 18 Kohlenstoffatomen, durch Sauerstoff oder Schwefel unterbrochenes Alkyl mit 2 bis 18 Kohlenstoffatomen, Dialkylaminoalkyl mit insgesamt 3 bis 16 Kohlenstoffatomen, Cyclopentyl, Cyclohexyl, Phenyl oder durch 1 bis 3 Alkylreste mit zusammen höchstens 18 Kohlenstoffatomen substituiertes Phenyl ist;
s 0, 1 oder 2 ist; die Substituenten $R'_{17}$ unabhängig voneinander Wasserstoff, Alkyl mit 1 bis 18 Kohlenstoffatomen, Cyclopentyl, Cyclohexyl, Phenyl, durch 1 oder 2 Alkylreste mit zusammen höchstens 16 Kohlenstoffatomen substituiertes Phenyl, ein Rest der Formel $-C_2H_4OH$, $-C_2H_4-O-C_tH_{2t+1}$ oder

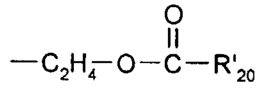

sind oder zusammen mit dem Stickstoffatom, an das sie gebunden sind, einen Piperidin- oder Morpholinrest bilden;
t 1 bis 18;
$R'_{20}$ Wasserstoff, Alkyl mit 1 bis 22 Kohlenstoffatomen oder Cycloalkyl mit 5 bis 12 Kohlenstoffatomen;
A ein gegebenenfalls durch Stickstoff, Sauerstoff oder Schwefel unterbrochenes Alkylen mit 2 bis 22 Kohlenstoffatomen;
$R'_{18}$ Wasserstoff, Alkyl mit 1 bis 18 Kohlenstoffatomen, Cyclopentyl, Cyclohexyl, Phenyl, durch 1 oder 2 Alkylreste mit zusammen höchstens 16 Kohlenstoffatomen substituiertes Phenyl oder Benzyl;

R'$_{19}$ Alkyl mit 1 bis 18 Kohlenstoffatomen bedeutet;
D -O-, -S-, -SO-, -$SO_2$- oder -C(R'$_{21}$)$_2$- ist;
die Substituenten R'$_{21}$ unabhängig voneinander Wasserstoff, $C_1$-$C_{16}$-Alkyl sind, wobei die beiden R'$_{21}$ zusammen 1 bis 16 Kohlenstoffatome enthalten, R'$_{21}$ ferner Phenyl oder einen Rest der Formel

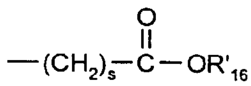 oder 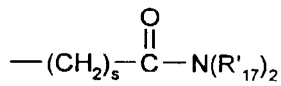

ist, worin s, R'$_{16}$ und
R'$_{17}$ die oben angegebenen Bedeutungen haben;
E ein Rest der Formel

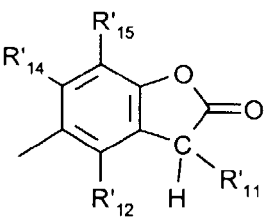

worin R'$_{11}$, R'$_{12}$ und R'$_{14}$ die oben angegebenen Bedeutungen haben; und
R'$_{15}$ Wasserstoff, Alkyl mit 1 bis 20 Kohlenstoffatomen, Cyclopentyl, Cyclohexyl, Chlor oder ein Rest der Formel

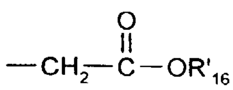 oder 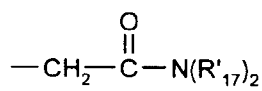

ist, worin R'$_{16}$ und R'$_{17}$ die oben angegebenen Bedeutungen haben, oder R'$_{15}$ zusammen mit R'$_{14}$ einen Tetramethylenrest bildet.

Bevorzugt sind solche Benzofuran-2-one, in denen R'$_{13}$ Wasserstoff, Alkyl mit 1 bis 12 Kohlenstoffatomen, Cyclopentyl, Cyclohexyl, Chlor oder ein Rest der Formel

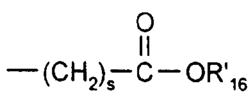 , 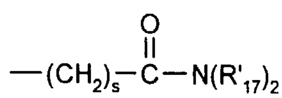

oder -D-E ist, worin s, R'$_{16}$, R'$_{17}$, D und E die oben angegebenen Bedeutungen haben, R'$_{16}$ insbesondere die Bedeutung von Wasserstoff, Alkyl mit 1 bis 18Kohlenstoffatomen, Cyclopentyl oder Cyclohexyl hat.

Bevorzugt sind weiterhin solche Benzofuran-2-one, in denen R'$_{11}$ Phenyl oder durch 1 oder 2 Alkylreste mit zusammen höchstens 12 Kohlenstoffatomen substituiertes Phenyl ist; R'$_{12}$ Wasserstoff; R'$_{14}$ Wasserstoff oder Alkyl mit 1 bis 12 Kohlenstoffatomen ist;
R'$_{13}$ Wasserstoff, Alkyl mit 1 bis 12 Kohlenstoffatomen,

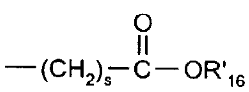 ,

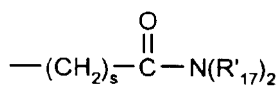

oder -D-E; $R'_{15}$ Wasserstoff, Alkyl mit 1 bis 20 Kohlenstoffatomen,

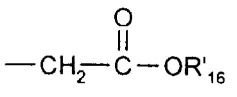 oder 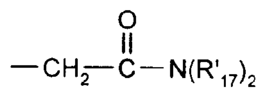

ist oder $R'_{15}$ zusammen mit $R'_{14}$ einen Tetramethylenrest bildet, wobei s, $R'_{16}$, $R'_{17}$, D und E die zu Anfang angegebenen Bedeutungen haben.

Ebenfalls von besonderem Interesse sind solche Benzofuran-2-one, in denen $R'_{13}$ Wasserstoff, Alkyl mit 1 bis 12 Kohlenstoffatomen oder -D-E ist; $R'_{12}$ und $R'_{14}$ unabhängig voneinander Wasserstoff oder Alkyl mit 1 bis 4 Kohlenstoffatomen sind; und $R'_{15}$ Alkyl mit 1 bis 20 Kohlenstoffatomen ist, wobei D und E die zu Anfang angegebenen Bedeutungen haben.

Ebenfalls von hervorgehobenem Interesse sind schliesslich solche Benzofuran-2-one, in denen $R'_{13}$ Alkyl mit 1 bis 4 Kohlenstoffatomen oder -D-E ist; $R'_{12}$ und $R'_{14}$ Wasserstoff sind; und $R'_{15}$ Alkyl mit 1 bis 4 Kohlenstoffatomen, Cyclopentyl oder Cyclohexyl ist, wobei D eine Gruppe $-C(R'_{21})_2-$ und E ein Rest der Formel

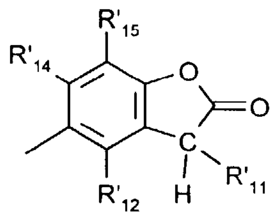

ist, wobei die Substituenten $R'_{21}$ gleich oder verschieden voneinander sind und je Alkyl mit 1 bis 4 Kohlenstoffatomen sind, und $R'_{11}$, $R'_{12}$, $R'_{14}$ und $R'_{15}$ die angegebene Bedeutung haben.

Die Menge an zusätzlich eingesetzten Benzofuran-2-onen, kann in weiten Grenzen schwanken. Beispielsweise können sie zu 0,0001 bis 5, vorzugsweise 0,001 bis 2, insbesondere 0,01 bis 2 Gew.-%, in den erfindungsgemäßen Zusammensetzungen enthalten sein.

Die Einarbeitung der Verbindungen der Formel I sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel I können auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden.

Die Verbindungen der Formel I können in reiner Form oder in Wachsen, Oelen oder Polymeren verkapselt in das zu stabilisierende Material eingearbeitet werden.

Die Verbindungen der Formel I können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel I, gegebenenfalls zusammen mit anderen Additiven, durch Aufsprühen zu applizieren.

Die so stabilisierten Materialien können in verschiedenster Form angewendet werden, z.B. als Folien, Fasern, Bändchen, Formmassen, Profile oder als Bindemittel für Lacke, insbesondere Pulverlacke, Klebstoffe oder Kitte.

Wie bereits erwähnt, handelt es sich bei den zu schützenden organischen Materialien vorzugsweise um organische, besonders synthetische, Polymere. Besonders vorteilhaft werden dabei thermoplastische Materialien geschützt. Vor allem ist dabei die ausgezeichnete Wirksamkeit der erfindungsgemässen Verbindungen der Formel I als Verarbeitungsstabilisatoren (Hitzestabilisatoren) hervorzuheben. Zu diesem Zweck werden sie vorteilhaft vor oder während der Verarbeitung des Polymeren diesem zugesetzt. Aber auch weitere Polymere (z.B. Elastomere) oder Schmierstoffe bzw. Hydraulikflüssigkeiten können gegen Abbau, z.B. lichtinduzierten oder thermooxidativen Abbau, stabilisiert werden. Elastomere sind der obigen Aufzählung von möglichen organischen Materialien zu entnehmen.

Die in Frage kommenden Schmierstoffe und Hydraulikflüssigkeiten basieren beispielsweise auf mineralischen

oder synthetischen Ölen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd.13, Seiten 85-94 (Verlag Chemie, Weinheim, 1977) beschrieben.

Eine bevorzugte Ausführungsform der vorliegenden Erfindung ist daher die Verwendung der Verbindungen der Formel I als Stabilisatoren, insbesondere Verarbeitungsstabilisatoren (Thermostabilisatoren), für organische Materialien, insbesondere thermoplastische Polymere, gegen oxidativen, thermischen oder lichtinduzierten Abbau.

Die erfindungsgemässen Verbindungen der Formel I zeichnen sich durch eine ausgeprägt gute Hydrolysestabilität und ein vorteilhaftes Farbverhalten, d.h. geringe Verfärbung der organischen Materialien während der Verarbeitung, aus.

Organische Materialien, die mit den Verbindungen der vorliegenden Erfindung stabilisiert sind, sind besonders gut vor einem lichtinduzierten Abbau geschützt.

Die vorliegende Erfindung betrifft auch ein Verfahren zum Stabilisieren eines organischen Materials gegen oxidativen, thermischen oder lichtinduzierten Abbau, dadurch gekennzeichnet, daß man diesem mindestens eine Verbindung der Formel I einverleibt oder auf dieses aufbringt.

Die folgenden Beispiele erläutern die Erfindung weiter. Angaben in Teilen oder Prozenten beziehen sich auf das Gewicht.

Beispiel 1: Herstellung der Verbindungen der Formel III (Tabelle 1).

a) Herstellung der Verbindung (101) (Tabelle 1).

Eine Mischung von 82,5 g (0,40 Mol) 2,4-Di-tert-butyl-phenol und 16,8 g (0,20 Mol) Cyclopentanon wird zusammengeschmolzen und anschliessend bei 40-45°C unter Rühren mit Salzsäure-Gas gesättigt. Nach 22 Stunden wird die tiefbraune Reaktionsmasse mit Hexan verdünnt und mit Wasser und Kochsalzlösung gewaschen. Die organische Phase wird über Magnesiumsulfat getrocknet und am Vakuumrotationsverdampfer eingeengt. Vakuumdestillation des Rückstandes liefert 11,5 g (21 %) 2,4-Di-tert-butyl-6-cyclopenten-1yl-phenol, Sdp. 98-112°C/ 0.08 mBar, gelbliches Oel (Verbindung (101), Tabelle 1).

b) Herstellung der Verbindung (102) (Tabelle 1).

24,75 g (120 mMol) 2,4-Di-tert-butylphenol und 2,95 g (30,0 mMol) Cyclohexanon werden bei 60°C eingeschmolzen. Anschliessend wird die Schmelze auf 45°C abgekühlt, mit Salzsäure-Gas gesättigt und während 24 Stunden bei 45°C gerührt. Das Reaktionsgemisch wird mit 50 ml Methanol versetzt und mit Eis/Wasser gekühlt. Das ausgefallene Produkt wird filtriert und mit wenig kaltem Methanol gewaschen. Es resultieren 4,55 g des Produktes. Das Filtrat wird am Vakuumrotationsverdampfer eingeengt und das überschüssige 2,4-Di-tert-butylphenol am Hochvakuum abdestilliert. Kristallisation des Rückstandes aus 10 ml Methanol liefert erneut 1,75 g des Produkts. Es werden somit total 6,3 g (73 %) 2,4-Di-tert-butyl-6-cyclohexen-1-yl-phenol, Smp. 102-104°C (Verbindung (101), Tabelle 1) erhalten.

c) Herstellung der Verbindung (103) (Tabelle 1).

Eine Mischung von 21,9 g (0,106 Mol) 2,4-Di-tert-butyl-phenol, 5,3 g (0,053 Mol) Tetrahydro-4H-pyran-4-on und 5 ml Essigsäure wird zusammengeschmolzen und dann bei 40-45°C unter Rühren mit Salzsäure-Gas gesättigt. Nach 60 Stunden Stehen bei Raumtemperatur wird die Reaktionsmasse mit Toluol verdünnt und mit Wasser, Kochsalz- und Natriumbicarbonatlösung gewaschen. Die organische Phase wird über Magnesiumsulfat getrocknet und am Rotationsverdampfer eingeengt. Kristallisation des Rückstandes aus Methanol liefert 7,2g (47 %) 2,4-Di-tert-butyl-6-(3,6-dihydro-2H-pyran-4-yl)-phenol, Smp. 135-139°C (Verbindung (103), Tabelle 1).

d) Herstellung der Verbindung (104) (Tabelle 1).

Eine Mischung von 17 g (0,082 Mol) 2,4-Di-tert-butyl-phenol, 4,8 g (0,041 Mol) Tetrahydrothiopyran-4-on und 5 ml Essigsäure wird zusammengeschmolzen und dann bei 40-45°C unter Rühren mit Salzsäure-Gas gesättigt. Nach 12 Stunden Rühren bei Raumtemperatur wird die Reaktionsmasse mit Toluol verdünnt und mit Wasser, Kochsalz- und Natriumbicarbonat-Lösung gewaschen. Die organische Phase wird über Magnesiumsulfat getrocknet und am Rotationsverdampfer eingeengt. Kristallisation des Rückstandes aus Methanol liefert 9,2 g (74 %) 2,4-Di-tert-butyl-6-(3,6-dihydro-2H-thiopyran-4-yl)-phenol, Smp. 115-119°C (Verbindung (104), Tabelle 1).

e) Herstellung der Verbindung (105) (Tabelle 1).

Eine Mischung von 82,5 g (0,40 Mol) 2,4-Di-tert-butyl-phenol und 22,5 g (0,20 Mol) Cycloheptanon wird zusammengeschmolzen und dann bei 35-40°C unter Rühren mit Salzsäure-Gas gesättigt. Nach 22 Stunden wird

das Reaktionsgemisch erneut mit Salzsäure-Gas gesättigt und anschliessend noch 17 Stunden gerührt. Die Reaktionsmasse wird danach mit Hexan verdünnt und mit Wasser und Kochsalzlösung gewaschen. Die organische Phase wird über Magnesiumsulfat getrocknet und am Rotationsverdampfer eingeengt. Chromatographie des Rückstandes an Kieselgel mit dem Laufmittelsystem Hexan/Essigsäureethylester = 99:1 und Kristallisation der reinen Fraktionen aus Ethanol liefert 5,4 g (9 %) 2,4-Ditert-butyl-6-cyclohepten-1-yl-phenol, Smp. 78-80°C (Verbindung (105), Tabelle 1).

f) Herstellung der Verbindung (106) (Tabelle 1).

Eine Mischung von 30,9 g (0,15 Mol) 2,4-Di-tert-butyl-phenol und 10,5 g (0,075 Mol) 3,3,5-Trimethyl-cyclohexanon wird zusammengeschmolzen und dann bei 35°C unter Rühren mit Salzsäure-Gas gesättigt. Nach 24 Stunden wird das Reaktionsgemisch erneut mit Salzsäure-Gas gesättigt und anschliessend noch 20 Stunden gerührt. Die grüne Reaktionsmasse wird dann mit Hexan verdünnt und mit Wasser und Kochsalzlösung gewaschen. Die organische Phase wird über Magnesiumsulfat getrocknet und am Rotationsverdampfer eingeengt. Chromatographie des Rückstandes an Kieselgel mit dem Laufmittelsystem Hexan/Essigsäureethylester = 49:1 liefert 10,6 g (43 %) 2,4-Di-tert-butyl-6-(3,5,5-trimethyl-cyclohexen-1-yl)-phenol als dickes, gelbliches Oel (Verbindung (106), Tabelle 1).

g) Die Herstellung der Verbindung (107), Tabelle 1, ist in EP-A-0 705 870, Beispiel 2, offenbart.

h) Die Herstellung der Verbindung (108), Tabelle 1, ist in U.S. 5,414,033, Beispiel 1, offenbart.

i) Herstellung der Verbindung (109) (Tabelle 1).

In 100 ml Xylol wird unter einer Stickstoffatmosphäre 15,0g ( 0,10 Mol) 4-tert-Butyl-phenol und 7,2 g (0,035 Mol) Aluminiumisopropylat vorgelegt. Danach wird zwecks Entfernung des gebildeten Isopropanols 30 ml Lösungsmittel abdestilliert. Das Reaktionsgemisch wird auf 100°C erwärmt und mit 9,8 g (0,10 Mol) Cyclohexanon versetzt und anschliessend während 20 Stunden unter Rückfluss gekocht. Das Reaktionsgemisch wird abgekühlt, mit 40 ml Wasser und 10 ml konzentrierter Salzsäure versetzt. Die organische Phase wird abgetrennt, mit Wasser gewaschen, über Magnesiumsulfat getrocknet und am Rotationsverdampfer eingeengt. Chromatographie des Rückstandes an Kieselgel mit dem Laufmittelsystem Hexan/Essigsäureethylester = 19:1 und Kristallisation der reinen Fraktionen aus Methanol liefert 6,3 g (27 %) 4-tert-butyl-2-cyclohexen-1-yl-phenol, Smp. 90-93°C (Verbindung (109), Tabelle 1).

j) Herstellung der Verbindung (110) (Tabelle 1).

Eine Mischung von 66,1 g (0,20 Mol) 2,4-Bis(1-methyl-1-phenyl-ethyl)-phenol und 9,8 g (0,10 Mol) Cyclohexanon wird zusammengeschmolzen und dann bei 40-45°C unter Rühren mit Salzsäure-Gas gesättigt. Nach 19 Stunden wird nochmals 9,8 g (0,10 Mol) Cyclohexanon zugegeben. Das Reaktionsgemisch wird erneut mit Salzsäure-Gas gesättigt und anschliessend noch 21 Stunden gerührt. Die Reaktionsmasse wird mit Petrolether verdünnt und mit Wasser gewaschen. Die organische Phase wird über Magnesiumsulfat getrocknet und am Rotationsverdampfer eingeengt. Chromatographie des Rückstandes an Kieselgel mit dem Laufmittelsystem Hexan/Essigsäureethylester = 39:1 liefert 1,3 g (2 %) 2-Cyclohexen-1-yl-4,6-bis(1-methyl-1-phenyl-ethyl)-phenol als dickes, gelbliches Oel (Verbindung (110), Tabelle 1).

k) Herstellung der Verbindung (111) (Tabelle 1).

Eine Mischung von 36,1 g (0,20 Mol) 3-tert-Butyl-4-hydroxy-anisol, 19,6 g (0,20 Mol) Cyclohexanon und 10 ml Essigsäure wird vorgelegt und unter Rühren bei Raumtemperatur mit Salzsäure-Gas gesättigt. Nach 64 Stunden Stehen bei Raumtemperatur wird nochmals mit Salzsäure-Gas nachgesättigt und weitere 5 Stunden bei 40°C nachgerührt. Das Reaktionsgemisch wird mit Hexan verdünnt, mit Wasser, Kochsalz- und Natriumbicarbonat-Lösung gewaschen. Die organische Phase wird über Magnesiumsulfat getrocknet und am Rotationsverdampfer eingeengt. Chromatographie des Rückstandes an Kieselgel mit dem Laufmittelsystem Essigsäureethylester/Hexan = 1:49 liefert 15,1 g (29 %) 2-tert-Butyl-6-cyclohexen-1-yl-4-methoxy-phenol als gelbliches, dickes Oel, $^1$H-NMR ($CDCl_3$), δ (ppm): 3,75 (s, 3H, $OCH_3$), 5,87 (m, 1H, Cyclohexenyl-H), (Verbindung (111), Tabelle 1).

Tabelle 1:

| Nr. | Verbindung | Smp. (°C) | Ausbeute |
|---|---|---|---|
| 101 | 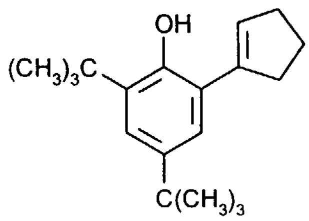 | 98-112°C/0,08 mBar (Siedepunkt) | 21 % |
| 102 | 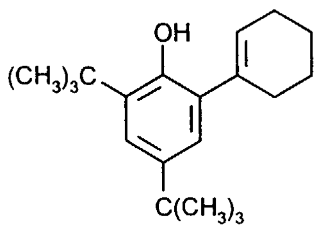 | 102-104 | 73 % |
| 103 | 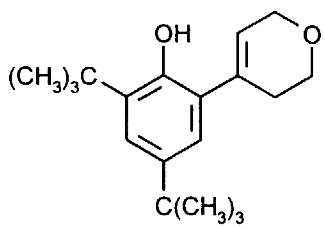 | 135-139 | 47 % |
| 104 | 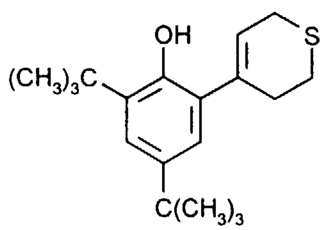 | 115-119 | 74 % |
| 105 | 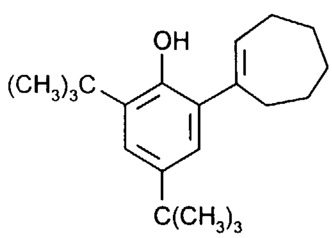 | 78-80 | 9 % |

Tabelle 1: (Fortsetzung)

| Nr. | Verbindung | Smp. (°C) | Ausbeute |
|---|---|---|---|
| 106 | 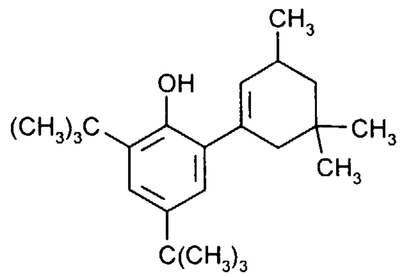 | Oel | 43 % |
| 107 | 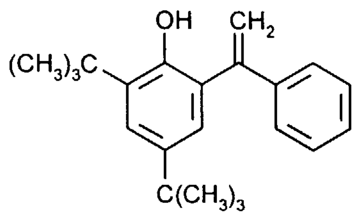 | Oel | 68 % |
| 108 | 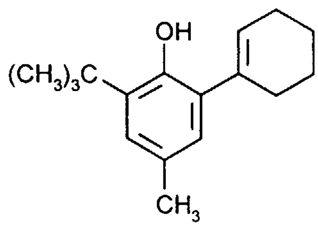 | Oel | 53 % |
| 109 | 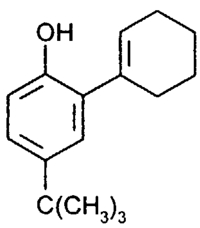 | 90-93 | 27 % |
| 110 | 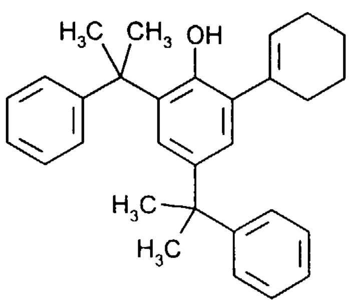 | Oel | 2 % |

Tabelle 1: (Fortsetzung)

| Nr. | Verbindung | Smp. (°C) | Ausbeute |
|---|---|---|---|
| 111 | 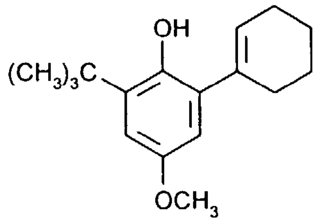 | Oel | 43 % |

Beispiel 2: Herstellung der Verbindungen der Formel IV (Tabelle 2).

a) Herstellung der Verbindung (202) (Tabelle 2).

Eine Mischung von 28,7 g (0,10 Mol) 2,4-Di-tert-butyl-6-cyclohexen-1-yl-phenol (Verbindung 102, Beispiel 1b) und 17,5 ml (0,20 Mol) Phosphortrichlorid wird unter Stickstoffatmosphäre während 5 Stunden unter Rückfluss gekocht. Das überschüssige Phosphortrichlorid wird abdestilliert und der Rückstand anschliessend im Vakuum bei 165-167°C/ 0,01 mBar destilliert. Es resultiert 30,8 g (88 %) 6,8-Di-tert-butyl-10-chloro-2,3,4,10-tetrahydro-1H-9-oxa-10-phospha-phenanthren, farblose, dicke, beim Stehen langsam kristallisierende Flüssigkeit, $^{31}P$-NMR ($CDCl_3$): $\delta$ = 130,5 ppm (s); $^{1}H$-NMR ($CDCl_3$): $\delta$ (ppm): 1,34 (s, 9H, $(CH_3)_3C$-), 1,44 (s, 9H, $(CH_3)_3C$-), (Verbindung (202), Tabelle 2).

In Analogie zu Beispiel 2a werden ausgehend von den in den Beispielen 1a, 1c, 1d, 1e, 1f, 1g, 1h, 1i, 1j und 1k beschriebenen Phenole (101), (103), (104), (105), (106), (107), (108), (109), (110) und (111) mit Phosphortrichlorid die Verbindungen (201), (203), (204), (205), (206), (207), (208), (209), (210) und (211) hergestellt (Tabelle 2).

Tabelle 2:

| Nr. | Verbindung | Smp. (°C) |
|---|---|---|
| 201 | 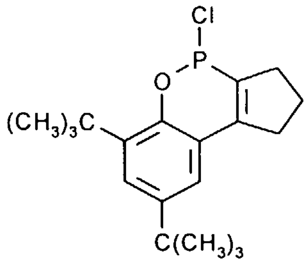 | Harz |
| 202 | 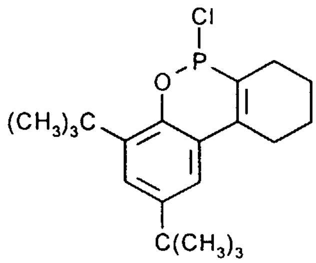 | Harz |
| 203 | 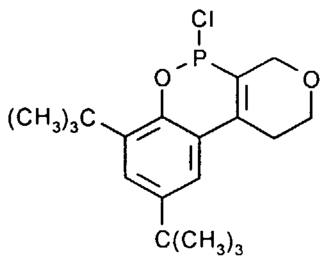 | Harz |
| 204 | 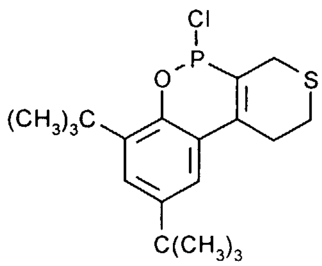 | Harz |
| 205 | 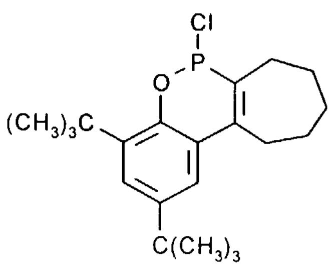 | Harz |

Tabelle 2: (Fortsetzung)

| Nr. | Verbindung | Smp. (°C) |
|---|---|---|
| 206 | 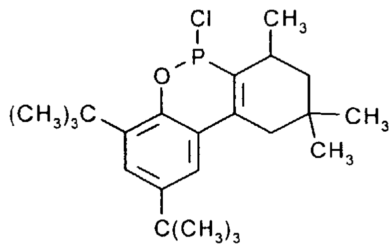 | Harz |
| 207 | 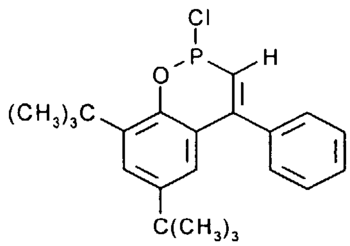 | Harz |
| 208 | 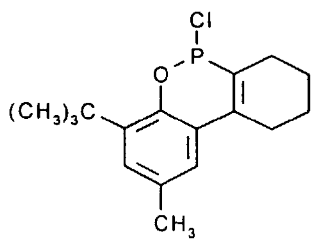 | Harz |
| 209 | 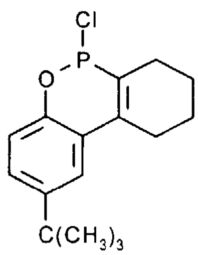 | Harz |
| 210 | 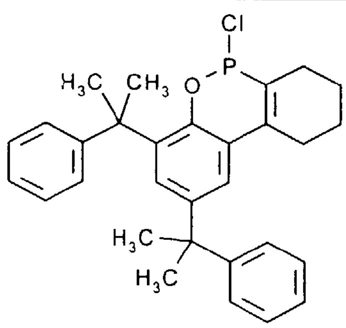 | Harz |

Tabelle 2: (Fortsetzung)

| Nr. | Verbindung | Smp. (°C) |
|---|---|---|
| 211 | 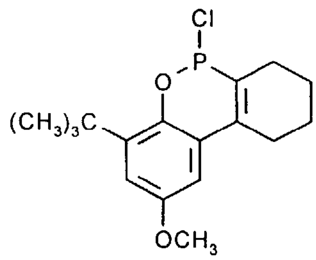 | Harz |

Beispiel 3: Herstellung der Verbindungen der Formel (Tabelle 3).

a) Herstellung der Verbindung (301) (Tabelle 3).

Eine Mischung von 2,74 g (0,01 Mol) 2,4-Di-tert-butyl-6-cyclopenten-1-yl-phenol (Verbindung (101), Beispiel 1a) und 2,7 ml (0,031 Mol) Phosphortrichlorid wird unter Stickstoffatmosphäre während 17 Stunden unter Rückfluss gekocht. Das Reaktionsgemisch wird anschliessend auf 100°C abgekühlt, mit 30 ml Toluol verdünnt und vorsichtig mit 20 ml Wasser während 10 Minuten bei 100°C hydrolysiert. Die organische Phase wird abgetrennt, mit Wasser gewaschen, über Natriumsulfat getrocknet und am Rotationsverdampfer eingeengt. Kristallisation des Rückstandes aus Petrolether/Toluol liefert 1,6 g (50 %) der Verbindung (301), Tabelle 3, Smp. 172-174°C. Analyse berechnet: C 71,67; H 8,55 %. gefunden: C 71,64; H 8,71 %.

b) Herstellung der Verbindung (302) (Tabelle 3).

Eine Mischung von 114,4 g (0,40 Mol) 2,4-Di-tert-butyl-6-cyclohexen-1-yl-phenol (Verbindung (102), Beispiel 1b) und 52,5 ml (0,60 Mol) Phosphortrichlorid wird unter Stickstoffatmosphäre während 4 Stunden unter Rückfluss gekocht. Das Reaktionsgemisch wird anschliessend auf 100°C abgekühlt, vorsichtig mit 100 ml Wasser hydrolysiert, 10 Minuten bei 100°C nachgerührt und auf 0°C abgekühlt. Das ausgefallene Produkt wird abfiltriert, mit Wasser neutral gewaschenen und im Vakuum bei 60°C getrocknet. Kristallisation aus Petrolether liefert 119 g (90 %) der Verbindung (302), Tabelle 3, Smp. 164-166°C. Analyse berechnet: C 72,26; H 8,79 %. gefunden: C 72,31; H 8,78 %.

c) Herstellung der Verbindung (303) (Tabelle 3).

Eine Mischung von 5,8 g (0,02 Mol) 2,4-Di-tert-butyl-6-(3,6-dihydro-2H-pyran-4-yl)-phenol (Verbindung (103), Beispiel 1c) und 5,2 ml (0,06 Mol) Phosphortrichlorid wird unter Stickstoffatmosphäre während 23 Stunden unter Rückfluss gekocht. Das Reaktionsgemisch wird anschliessend auf 100°C abgekühlt, mit 50 ml Petrolether verdünnt und vorsichtig mit 20 ml Wasser versetzt und während 15 Minuten bei 100°C hydrolysiert. Das Reaktionsgemisch wird abgekühlt und mit Essigsäureethylester verdünnt. Die organische Phase wird mit Wasser gewaschen, über Natriumsulfat getrocknet und am Rotationsverdampfer eingeengt. Kristallisation des Rückstandes aus Petrolether liefert 5,6 g (84 %) der Verbindung (303), Tabelle 3, Smp. 156-157°C. Analyse berechnet: C 68,25; H 8,14 %. gefunden: C 68,24; H 8,06 %.

d) Herstellung der Verbindung (304) (Tabelle 3).

Eine Mischung von 6,1 g (0,02 Mol) 2,4-Di-tert-butyl-6-(3,6-dihydro-2H-thiopyran-4-yl)-phenol (Verbindung (104), Beispiel 1d) und 2,6 ml (0,03 Mol) Phosphortrichlorid wird unter Stickstoffatmosphäre während 14,5 Stunden unter Rückfluss gekocht. Das Reaktionsgemisch wird anschliessend auf 100°C abgekühlt, mit 50 ml Petrolether verdünnt, vorsichtig mit 20 ml Wasser versetzt und während 15 Minuten bei 100°C hydrolysiert. Das Reaktionsgemisch wird abgekühlt. Die organische Phase wird mit Wasser gewaschen, über Natriumsulfat getrocknet und am Rotationsverdampfer eingeent. Kristallisation des Rückstandes aus Petrolether liefert 6,5 g (93 %) der Verbindung (304), Tabelle 3, Smp. 177-179°C. Analyse berechnet: C 65,12; H 7,77; S 9,15 %. gefunden: C 65,11; H 7,72; S 9,14 %.

e) Herstellung der Verbindung (305) (Tabelle 3).

Eine Mischung von 3,0 g (0,01 Mol) 2,4-Di-tert-butyl-6-cyclohepten-1-yl-phenol (Verbindung (105), Beispiel

1e) und 2,6 ml (0,03 Mol) Phosphortrichlorid wird unter Stickstoffatmosphäre während 22 Stunden unter Rückfluss gekocht. Das Reaktionsgemisch wird anschliessed auf 100°C abgekühlt, mit 20 ml Toluol verdünnt, vorsichtig mit 20 ml Wasser versetzt und während 15 Minuten bei 100°C hydrolysiert. Die organische Phase wird mit Wasser gewaschen, über Natriumsulfat getrocknet und am Rotationsverdampfer eingeengt. Kristallisation des Rückstandes aus Hexan liefert 2,8 g (81 %) der Verbindung (305), Tabelle 3, Smp. 148-150°C. Analyse berechnet: C 72,80; H 9,02 %. gefunden: C 72,80; H 8,93 %.

f) Herstellung der Verbindung (306) (Tabelle 3).

Eine Mischung von 5,1 g (0,0155 Mol) 2,4-Di-tert-butyl-6-(3,5,5-trimethyl-cyclohexen-1-yl)-phenol (Verbindung (106), Beispiel 1f) und 4,2 ml (0,048 Mol) Phosphortrichlorid wird unter Stickstoffatmosphäre während 23,5 Stunden unter Rückfluss gekocht. Das Reaktionsgemisch wird anschliessend auf 100°C abgekühlt, mit 20 ml Toluol verdünnt, vorsichtig mit 10 ml Wasser versetzt und während 15 Minuten bei 100°C hydrolysiert. Die organische Phase wird mit Wasser gewaschen, über Natriumsulfat getrocknet und am Rotationsverdampfer eingeengt. Chromatographie des Rückstandes an Kieselgel mit dem Laufmittelsystem Essigsäureethylester/Hexan = 1:2 liefert 4,55 g (78 %) der Verbindung (306), Tabelle 3, als farbloses, dickes Harz. Analyse berechnet: C 73,76; H 9,42 %. gefunden: C 73,74; H 9,38 %.

g) Herstellung der Verbindung (307) (Tabelle 3).

Eine Mischung von 5,25 g (0,017 Mol) Di-tert-butyl-6-(1-phenyl-vinyl)-phenol (Verbindung (107), Beispiel 1g) und 3 ml (0,034 Mol) Phosphortrichlorid wird unter Stickstoffatmosphäre während 15 Stunden unter Rückfluss gekocht. Das Reaktionsgemisch wird anschliessend auf 100°C abgekühlt, mit 50 ml Toluol verdünnt, vorsichtig mit 15 ml Wasser versetzt und während 15 Minuten bei 100°C hydrolysiert. Die organische Phase wird mit Wasser gewaschen, über Natriumsulfat getrocknet und am Rotationsverdampfer eingeengt. Kristallisation des Rückstandes aus Petrolether liefert 2,05 g (34 %) der Verbindung (307), Tabelle 3, Smp. 113°C. Analyse berechnet: C 74,55; H 7,68 %. gefunden: C 74,72; H 7,68 %.

h) Herstellung der Verbindung (308) (Tabelle 3).

Eine Mischung von 14,3 g (0,059 Mol) 2-tert-Butyl-6-cyclohexen-1-yl-4-methyl-phenol (Verbindung (108), Beispiel 1h) und 10 ml (0,11 Mol) Phosphortrichlorid wird unter Stickstoffatmosphäre während 16 Stunden unter Rückfluss gekocht. Das Reaktionsgemisch wird anschliessend in 700 ml Wasser gegossen und mit 200 ml Essigsäureethylester extrahiert. Die organische Phase wird mit Wasser gewaschen, über Natriumsulfat getrocknet und am Rotationsverdampfer eingeengt. Kristallisation des Rückstandes aus Petrolether liefert 14,0 g (82 %) der Verbindung (308), Tabelle 3, Smp. 157-159°C. Analyse berechnet: C 70,33; H 7,98 %. gefunden: C 70,36; H 7,93 %.

i) Herstellung der Verbindung (309) (Tabelle 3).

Eine Mischung von 2,3 g (0,01 Mol) 4-tert-Butyl-2-cyclohexen-1-yl-phenol (Verbindung (109), Beispiel 1i) und 2,7 ml (0,03 Mol) Phosphortrichlorid wird unter Stickstoffatmosphäre während 22 Stunden unter Rückfluss gekocht. Das Reaktionsgemisch wird anschliessend auf 100°C abgekühlt, mit 20 ml Toluol verdünnt, vorsichtig mit 10 ml Wasser versetzt und während 10 Minuten bei 100°C hydrolysiert. Die organische Phase wird mit Wasser gewaschen, über Natriumsulfat getrocknet und am Rotationsverdampfer eingeengt. Chromatographie des Rückstandes an Kieselgel mit Essigsäureethylester liefert 1,3 g (47 %) der Verbindung (309), Tabelle 3, amorphes Harz. Massenspektrum für $C_{16}H_{21}O_2P$ (MG = 276,3): $M^+$ = 276.

j) Herstellung der Verbindung (310) (Tabelle 3).

Eine Mischung von 1,1 g (2,70 mMol) 2-Cyclohexen-1-yl-4,6-bis(1-methyl-1-phenyl-ethyl)-phenol (Verbindung (110), Beispiel 1j), 1 ml (11,0 mMol) Phosphortrichlorid und 1ml Toluol wird unter Stickstoffatmosphäre während 22 Stunden unter Rückfluss gekocht. Das Reaktionsgemisch wird anschliessend auf 100°C abgekühlt, mit 10 ml Toluol verdünnt, vorsichtig mit 10 ml Wasser versetzt und während 30 Minuten bei 100°C hydrolysiert. Die organische Phase wird mit Wasser gewaschen, über Natriumsulfat getrocknet und am Rotationsverdampfer eingeengt. Chromatographie des Rückstandes an Kieselgel mit dem Laufmittelsystem Essigsäureethylester/Hexan = 3:4 liefert 0,2 g (16 %) der Verbindung (310), Tabelle 3, amorphes Harz. Massenspektrum für $C_{30}H_{33}O_2P$ (MG = 456,6): $M^+$ = 456.

k) Herstellung der Verbindung (311) (Tabelle 3).

Eine Mischung von 10,4 g (0,04 Mol) 2-tert-Butyl-6-cyclohexen-1-yl-4-methoxy-phenol und 5,25 ml (0,06 Mol) Phosphortrichlorid wird unter Stickstoffatmosphäre während 4,5 Stunden unter Rückfluss gekocht. Das Reaktionsgemisch wird anschliessend mit 20 ml Ligroin ( Sdp. 80-110°C) verdünnt, vorsichtig mit 10 ml Wasser versetzt und während 10 Minuten unter Rückfluss hydrolysiert. Das Reaktionsgemisch wird abgekühlt, der ausgefallene

Produkt filtriert und mit Wasser und Ligroin gewaschen. Kristallisation des Filterrückstandes aus Ligroin (Sdp. 110-140°C) ergibt 7,5 g (61%) der Verbindung (311), Tabelle 3, Smp. 123-126°C. Massenspektrum für $C_{17}H_{23}O_3P$ (MG = 306,3): $M^+$ = 306.

Tabelle 3:

| Nr. | Verbindung | Smp. (°C) | Ausbeute |
|---|---|---|---|
| 301 | 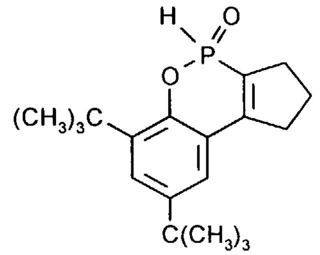 | 172-174 | 50 % |
| 302 | 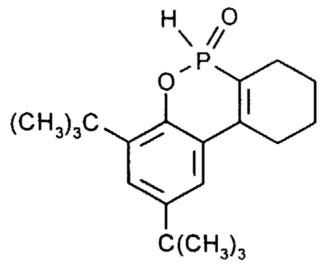 | 164-166 | 90 % |
| 303 | 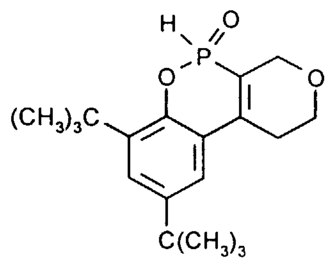 | 156-157 | 84 % |
| 304 | 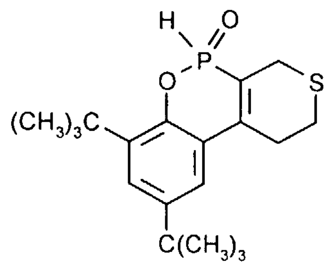 | 177-179 | 93 % |
| 305 | 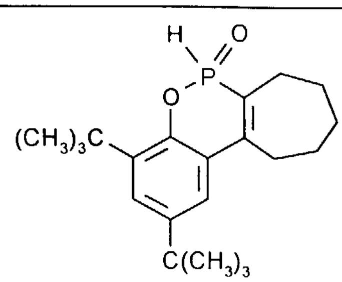 | 148-150 | 81 % |

Tabelle 3: (Fortsetzung)

| Nr. | Verbindung | Smp. (°C) | Ausbeute |
|---|---|---|---|
| 306 | 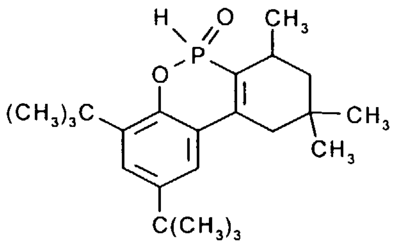 | Harz | 78 % |
| 307 | 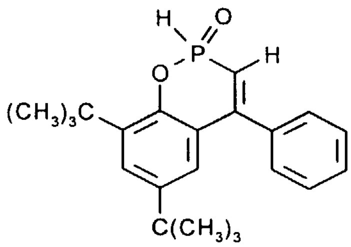 | 113 | 34 % |
| 308 | 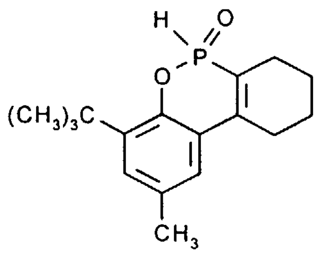 | 157-159 | 98 % |
| 309 | 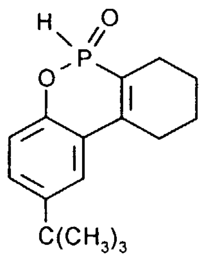 | amorph | 47 % |
| 310 | 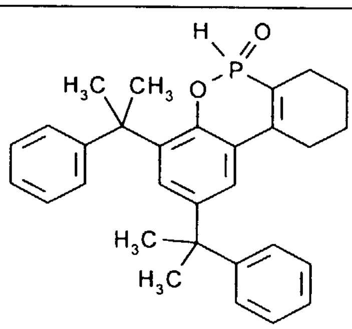 | amorph | 16 % |

Tabelle 3: (Fortsetzung)

| Nr. | Verbindung | Smp. (°C) | Ausbeute |
|---|---|---|---|
| 311 | 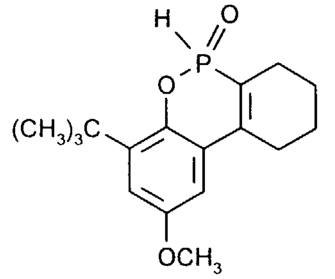 | 123-126 | 61 % |

Beispiel 4: Stabilisierung von ABS-Spritzguss gegen Vergilbung.

3 kg ABS Copolymer (Ronfalin®TZ 220 der Firma DSM) und 9 g (0,3 %) der Verbindung aus Tabelle 3 werden in einer 10 Liter Aluminiumflasche auf einem Rhönrad während 30 Minuten gemischt. Diese Mischung wird anschliessend in einem "Single Screw Extruder Schwabenthan" bei 220°C extrudiert. Der Polymerstrang wird granuliert. Aus dem so erhaltenen Granulat werden anschliessend mit einer Spritzgussmaschine vom Typ Engel EK, bei 230°C und einer Verweilzeit von 2 Minuten, Platten mit 60 mal 44 mal 2 mm Abmessung gespritzt. Von diesen Platten wird der Yellowness Index (YI) nach ASTM D 1925-70 bestimmt. Niedrige YI-Werte bedeuten wenig Verfärbung, hohe YI-Werte starke Verfärbung der Platten. Je geringer die Verfärbung, desto wirksamer ist der Stabilisator. Die Resultate sind in Tabelle 4 zusammengefasst.

Tabelle 4:

| Beispiel | Verbindung aus Tabelle 3 | Yellowness Index |
|---|---|---|
| 4a | — | 62 |
| 4b | 302 | 57 |

**Patentansprüche**

**1.** Verbindungen der Formel I

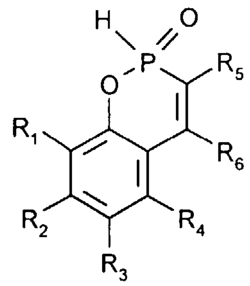

(I)

worin

$R_1$, $R_2$, $R_3$ und $R_4$ unabhängig voneinander Wasserstoff, Halogen, Hydroxy, $C_1$-$C_{25}$-Alkyl, durch Sauerstoff, Schwefel oder $>N-R_7$ unterbrochenes $C_2C_{25}$-Alkyl; $C_2$-$C_{24}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl; unsubstituiertes oder durch $C_1$-$C_4$-Alkyl substituiertes $C_5$-$C_{12}$-Cycloalkenyl; $C_1$-$C_{18}$-Alkoxy, $C_2$-$C_{24}$-Alkenyloxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoyl-

amino, $C_3$-$C_{25}$-Alkenoyloxy, durch Sauerstoff, Schwefel oder $>N-R_7$ unterbrochenes $C_3$-$C_{25}$-Alkanoyloxy; $C_5$-$C_9$-Cycloalkylcarbonyloxy, Benzoyloxy oder durch $C_1$-$C_{12}$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich $-(CH_2)_m-COR_8$ oder $-(CH_2)_nOR_9$ darstellt, oder wenn $R_2$ und $R_4$ Wasserstoff sind, $R_3$ zusätzlich einen Rest der Formel IIa, IIb oder IIc

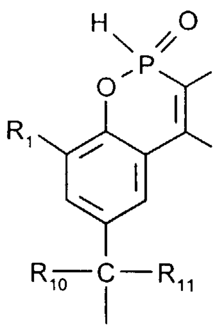

(IIa)

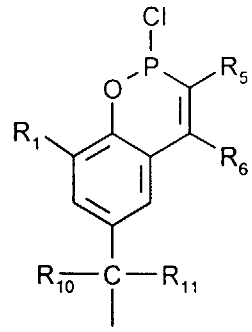

(IIb)

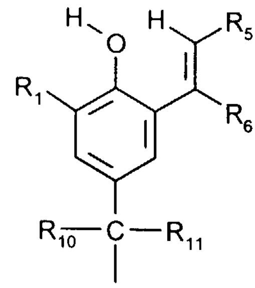

(IIc)

bedeutet,

$R_5$ und $R_6$ unabhängig voneinander Wasserstoff, $C_1$-$C_{25}$-Alkyl, durch Sauerstoff, Schwefel oder $>N-R_7$ unterbrochenes $C_2C_{25}$-Alkyl; $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl darstellen, oder $R_5$ und $R_6$ zusammen mit den Kohlenstoffatomen, an die sie gebunden sind, ein unsubstituiertes $C_5$-$C_{12}$-Cycloalkenylen oder ein durch Sauerstoff, Schwefel oder $>N-R_7$ unterbrochenes unsubstituiertes $C_4$-$C_{11}$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert sind,

$R_7$ Wasserstoff, $C_1$-$C_8$-Alkyl, $C_1$-$C_8$-Alkoxy, $C_1$-$C_8$-Alkanoyl oder unsubstituiertes oder mit $C_1$-$C_4$-Alkyl substituiertes Benzoyl bedeutet,

$R_8$ Hydroxy,

$\left[-O^{-}\ \frac{1}{r}M^{r+}\right]$, $C_1$-$C_{18}$-Alkoxy oder 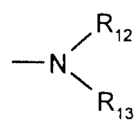

darstellt,

$R_9$ Wasserstoff, $C_1$-$C_8$-Alkanoyl oder unsubstituiertes oder mit $C_1$-$C_4$-Alkyl substituiertes Benzoyl bedeutet,

$R_{10}$ und $R_{11}$ unabhängig voneinander Wasserstoff, $CF_3$, $C_1$-$C_{12}$-Alkyl, unsubstituiertes oder durch $C_1$-$C_4$-Alkyl substituiertes Phenyl; oder $-(CH_2)_s-COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_{12}$-Cycloalkylidenring bilden,

$R_{12}$ und $R_{13}$ unabhängig voneinander Wasserstoff oder $C_1$-$C_{18}$-Alkyl darstellen,

$R_{14}$ Hydroxy, $C_1$-$C_{18}$-Alkoxy oder

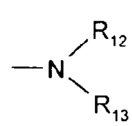

darstellt,

M ein r-wertiges Metallkation ist,

m 0, 1 oder 2 darstellt,
n 1, 2, 3, 4, 5 oder 6 bedeutet,
r 1, 2 oder 3 ist, und
s 0 oder 1 darstellt.

**2.** Verbindungen gemäss Anspruch 1, worin $R_2$ und $R_4$ Wasserstoff darstellen.

**3.** Verbindungen gemäss Anspruch 1, worin
$R_5$ und $R_6$ unabhängig voneinander Wasserstoff, $C_1$-$C_4$-Alkyl oder Phenyl darstellen, oder $R_5$ und $R_6$ zusammen mit den Kohlenstoffatomen, an die sie gebunden sind, ein unsubstituiertes $C_5$-$C_7$-Cycloalkenylen oder ein durch Sauerstoff oder Schwefel unterbrochenes unsubstituiertes $C_5$-$C_6$-Cycloalkenylen bilden, deren Ringe gegebenenfalls mit $C_1$-$C_4$-Alkyl oder Phenyl substituiert sind.

**4.** Verbindungen gemäss Anspruch 1, worin

$R_1$, $R_2$, $R_3$ und $R_4$ unabhängig voneinander Wasserstoff, Chlor, Hydroxy, $C_1$-$C_{18}$-Alkyl, durch Sauerstoff, Schwefel oder $>N-R_7$ unterbrochenes $C_2$-$C_{18}$-Alkyl; $C_2$-$C_{18}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes $C_5$-$C_8$-Cycloalkenyl; $C_1$-$C_{18}$-Alkoxy, $C_2$-$C_{18}$-Alkenyloxy, $C_1$-$C_{18}$-Alkylthio, $C_1$-$C_4$-Alkylamino, Di-($C_1$-$C_4$-alkyl)amino, $C_1$-$C_{18}$-Alkanoyloxy, $C_1$-$C_{18}$-Alkanoylamino, $C_3$-$C_{18}$-Alkenoyloxy, durch Sauerstoff, Schwefel oder $>N-R_7$ unterbrochenes $C_3$-$C_{18}$-Alkanoyloxy; $C_5$-$C_9$-Cycloalkylcarbonyloxy, Benzoyloxy oder durch $C_1$-$C_4$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich $-(CH_2)_m-COR_8$ oder $-(CH_2)_nOR_9$ darstellt, oder wenn $R_2$ und $R_4$ Wasserstoff sind, $R_3$ zusätzlich einen Rest der Formel IIa

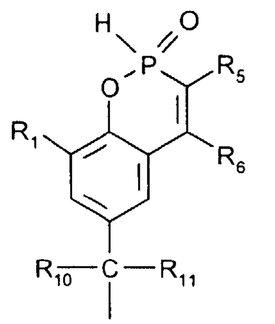

(IIa)

bedeutet,
$R_5$ und $R_6$ unabhängig voneinander Wasserstoff, $C_1$-$C_{18}$-Alkyl, durch Sauerstoff, Schwefel oder $>N-R_7$ unterbrochenes $C_2$-$C_{16}$-Alkyl; $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_8$-Cycloalkyl darstellen, oder $R_5$ und $R_6$ zusammen mit den Kohlenstoffatomen, an die sie gebunden sind, ein unsubstituiertes $C_5$-$C_9$-Cycloalkenylen oder ein durch Sauerstoff, Schwefel oder $>N-R_7$ unterbrochenes unsubstituiertes $C_4$-$C_8$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_8$-Cycloalkyl; unsubstituiertem oder mit $C_1$-$C_4$-Alkyl substituiertem Phenyl; oder mit Phenylen substituiert sind,
$R_7$ Wasserstoff, $C_1$-$C_6$-Alkyl, $C_1$-$C_6$-Alkoxy, $C_1$-$C_6$-Alkanoyl oder Benzoyl bedeutet,
$R_8$ Hydroxy,

$\left[-O^- \frac{1}{r} M^{r+}\right]$, $C_1$-$C_{12}$-Alkoxy oder 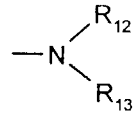

darstellt,
$R_9$ Wasserstoff, $C_1$-$C_8$-Alkanoyl oder Benzoyl bedeutet,
$R_{10}$ und $R_{11}$ unabhängig voneinander Wasserstoff, $CF_3$, $C_1$-$C_{10}$-Alkyl, unsubstituiertes oder durch $C_1$-$C_4$-Alkyl

substituiertes Phenyl; oder $-(CH_2)_s-COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_9$-Cycloalkylidenring bilden,
$R_{12}$ und $R_{13}$ unabhängig voneinander Wasserstoff oder $C_1$-$C_{12}$-Alkyl darstellen,
$R_{14}$ Hydroxy, $C_1$-$C_{12}$-Alkoxy oder

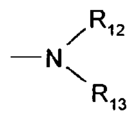

darstellt,
M ein Natrium, Kalium, Calcium oder Aluminium ist,
m 1 oder 2 darstellt,
n 2, 3, 4, 5 oder 6 bedeutet,
r 1, 2 oder 3 ist, und
s 0 oder 1 darstellt.

**5.** Verbindungen gemäss Anspruch 1, worin

$R_1$, $R_2$, $R_3$ und $R_4$ unabhängig voneinander Wasserstoff, Chlor, $C_1$-$C_{12}$-Alkyl, durch Sauerstoff oder Schwefel unterbrochenes $C_2$-$C_{12}$-Alkyl; $C_2$-$C_{12}$-Alkenyl, $C_7$-$C_9$-Phenylalkyl, Phenyl, $C_5$-$C_8$-Cycloalkyl, $C_5$-$C_8$-Cycloalkenyl, $C_1$-$C_{12}$-Alkoxy, $C_3$-$C_{12}$-Alkenyloxy, $C_1$-$C_{18}$-Alkylthio, $C_1$-$C_{12}$-Alkanoyloxy, $C_1$-$C_{12}$-Alkanoylamino, $C_3$-$C_{12}$-Alkenoyloxy, durch Sauerstoff oder Schwefel unterbrochenes $C_3$-$C_{12}$-Alkanoyloxy; $C_5$-$C_8$-Cycloalkylcarbonyloxy oder Benzoyloxy darstellen; $R_3$ zusätzlich einen Rest der Formel IIa

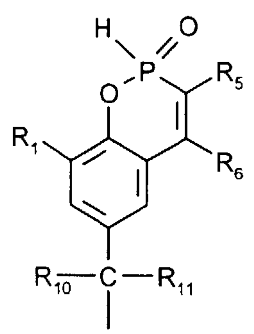

(IIa)

bedeutet,
$R_5$ und $R_6$ unabhängig voneinander Wasserstoff, $C_1$-$C_{12}$-Alkyl, durch Sauerstoff oder Schwefel unterbrochenes $C_2$-$C_{12}$-Alkyl; $C_7$-$C_9$-Phenylalkyl, Phenyl oder $C_5$-$C_8$-Cycloalkyl darstellen, oder $R_5$ und $R_6$ zusammen mit den Kohlenstoffatomen, an die sie gebunden sind, ein unsubstituiertes $C_5$-$C_9$-Cycloalkenylen oder ein durch Sauerstoff oder Schwefel unterbrochenes $C_4$-$C_8$-Cycloalkenylen bilden, deren Ringe gegebenenfalls mit $C_1$-$C_4$-Alkyl, $C_5$-$C_8$-Cycloalkyl oder Phenyl substituiert sind,
$R_{10}$ und $R_{11}$ unabhängig voneinander Wasserstoff, $CF_3$, $C_1$-$C_8$-Alkyl, Phenyl oder $-(CH_2)_s-COR_{14}$ bedeuten, oder $R_{10}$ und $R_{11}$ zusammen mit dem Kohlenstoffatom, an das sie gebunden sind, einen $C_5$-$C_9$-Cycloalkylidenring bilden,
$R_{14}$ Hydroxy oder $C_1$-$C_{12}$-Alkoxy darstellt, und
s 0 oder 1 bedeutet.

**6.** Verbindungen gemäss Anspruch 1, worin

$R_1$ Wasserstoff, $C_1$-$C_4$-Alkyl, Cyclohexyl, 1-Cyclohexenyl oder $C_7$-$C_9$-Phenylalkyl bedeutet,
$R_2$ Wasserstoff darstellt,
$R_3$ Wasserstoff, $C_1$-$C_4$-Alkyl, Cyclohexyl, 2-Cyclohexenyl, $C_7$-$C_9$-Phenylalkyl oder $C_1$-$C_4$-Alkoxy bedeutet,
$R_4$ Wasserstoff darstellt, und

$R_5$ und $R_6$ unabhängig voneinander Wasserstoff, $C_1$-$C_4$-Alkyl oder Phenyl darstellen, oder $R_5$ und $R_6$ zusammen mit den Kohlenstoffatomen, an die sie gebunden sind, ein unsubstituiertes $C_5$-$C_7$-Cycloalkenylen oder ein durch Sauerstoff oder Schwefel unterbrochenes unsubstituiertes $C_5$-$C_6$-Cycloalkenylen bilden, deren Ringe gegebenenfalls mit $C_1$-$C_4$-Alkyl oder Phenyl substituiert sind.

**7.** Verbindungen gemäss Anspruch 1, worin

$R_1$ Wasserstoff, $C_1$-$C_4$-Alkyl oder $C_7$-$C_9$-Phenylalkyl bedeutet,
$R_2$ Wasserstoff darstellt,
$R_3$ $C_1$-$C_4$-Alkyl, $C_7$-$C_9$-Phenylalkyl oder $C_1$-$C_4$-Alkoxy bedeutet,
$R_4$ Wasserstoff darstellt, und
$R_5$ und $R_6$ unabhängig voneinander Wasserstoff oder Phenyl darstellen, oder $R_5$ und $R_6$ zusammen mit den Kohlenstoffatomen, an die sie gebunden sind, ein unsubstituiertes $C_5$-$C_7$-Cycloalkenylen oder ein durch Sauerstoff oder Schwefel unterbrochenes unsubstituiertes $C_5$-Cycloalkenylen bilden, deren Ringe gegebenenfalls mit $C_1$-$C_4$-Alkyl substituiert sind.

**8.** Zusammensetzung enthaltend

a) ein dem oxidativen, thermischen oder lichtinduzierten Abbau unterworfenes organisches Material, und
b) mindestens eine Verbindung der Formel I gemäss Anspruch 1.

**9.** Zusammensetzung gemäss Anspruch 8, enthaltend neben den Komponenten (a) und (b) zusätzlich weitere Additive.

**10.** Zusammensetzung gemäss Anspruch 9, enthaltend als weitere Additive phenolische Antioxidantien, Lichtschutzmittel oder/und Verarbeitungsstabilisatoren.

**11.** Zusammensetzung gemäss Anspruch 9, enthaltend als weiteres Additiv mindestens eine Verbindung vom Typ der Benzofuran-2-one.

**12.** Zusammensetzung gemäss Anspruch 8, enthaltend als Komponente (a) natürliche, halbsynthetische oder synthetische Polymere.

**13.** Zusammensetzung gemäss Anspruch 8, enthaltend als Komponente (a) thermoplastische Polymere.

**14.** Zusammensetzung gemäss Anspruch 8, enthaltend als Komponente (a) ein Copolymer oder Pfropfcopolymer von Styrol oder $\alpha$-Methylstyrol mit Dienen, Polybutadien oder Acrylderivaten.

**15.** Zusammensetzung gemäss Anspruch 8, enthaltend als Komponente (a) ein ABS-Spritzguss.

**16.** Zusammensetzung gemäss Anspruch 8, worin die Komponente (b) in einer Menge von 0,01 bis 10 % bezogen auf das Gewicht der Komponente (a) vorliegt.

**17.** Verwendung der Verbindungen der Formel I gemäss Anspruch 1 als Stabilisatoren für organische Materialien gegen oxidativen, thermischen oder lichtinduzierten Abbau.

**18.** Verwendung gemäss Anspruch 17 als Verarbeitungsstabilisatoren (Thermostabilisatoren) in thermoplastischen Polymeren.

**19.** Verfahren zum Stabilisieren eines organischen Materials gegen oxidativen, thermischen oder lichtinduzierten Abbau, dadurch gekennzeichnet, dass man diesem mindestens eine Verbindung der Formel I gemäss Anspruch 1 einverleibt oder auf dieses aufbringt.

**20.** Verfahren zur Herstellung von Verbindungen der Formel I

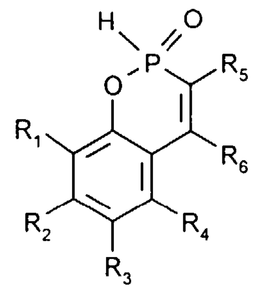

(I)

worin die allgemeinen Symbole wie in Anspruch 1 definiert sind, dadurch gekennzeichnet, dass eine Verbindung der Formel III

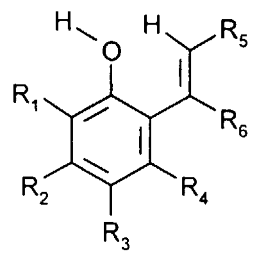

(III)

worin die allgemeinen Symbole wie in Anspruch 1 definiert sind, mit Phosphortrichlorid zu einer Verbindung der Formel IV

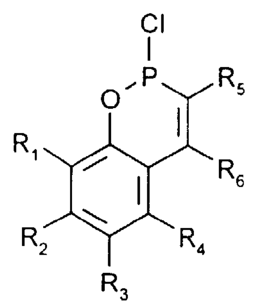

(IV)

worin die allgemeinen Symbole wie in Anspruch 1 definiert sind, umgesetzt wird, und diese anschliessend ohne Isolierung mit Wasser hydrolisiert wird.

**21.** Verbindungen der Formel IV

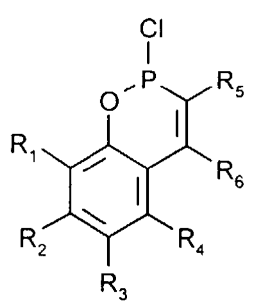

(IV)

worin die allgemeinen Symbole wie in Anspruch 1 definiert sind.

**22.** Verbindungen der Formel III

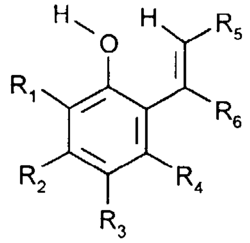

(III)

$R_1$, $R_2$, $R_3$ und $R_4$ unabhängig voneinander Wasserstoff, Halogen, Hydroxy, $C_1$-$C_{25}$-Alkyl, durch Sauerstoff, Schwefel oder $\rangle$N-$R_7$ unterbrochenes $C_2$-$C_{25}$-Alkyl; $C_2$-$C_{24}$-Alkenyl, $C_7$-$C_9$-Phenylalkyl, unsubstituiertes oder durch $C_1$-$C_4$-Alkyl substituiertes Phenyl; unsubstituiertes oder durch $C_1$-$C_4$-Alkyl substituiertes $C_5$-$C_{12}$-Cycloalkyl; unsubstituiertes oder durch $C_1$-$C_4$-Alkyl substituiertes $C_5$-$C_{12}$-Cycloalkenyl; $C_1$-$C_{18}$-Alkoxy, $C_2$-$C_{24}$-Alkenyloxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $\rangle$N-$R_7$ unterbrochenes $C_3$-$C_{25}$-Alkanoyloxy; $C_5$-$C_9$-Cycloalkylcarbonyloxy, Benzoyloxy oder durch $C_1$-$C_{12}$-Alkyl substituiertes Benzoyloxy darstellen; oder ferner die Reste $R_1$ und $R_2$ oder die Reste $R_2$ und $R_3$ oder die Reste $R_3$ und $R_4$ zusammen mit den Kohlenstoffatomen, an die sie gebunden sind, einen Benzoring bilden, $R_3$ zusätzlich -$(CH_2)_m$-$COR_8$ oder -$(CH_2)_nOR_9$ darstellt, oder wenn $R_2$ und $R_4$ Wasserstoff sind, $R_3$ zusätzlich einen Rest der Formel IIa, IIb oder IIc

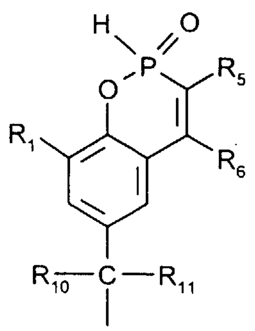

(IIa)

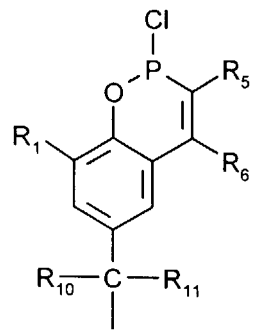

(IIb)

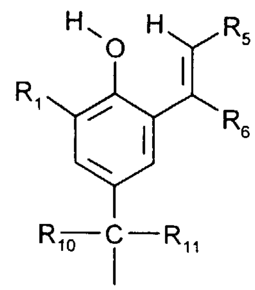

(IIc)

bedeutet,

$R_5$ und $R_6$ zusammen mit den Kohlenstoffatomen, an die sie gebunden sind, ein unsubstituiertes Cyclopentenylen oder $C_7$-$C_{12}$-Cycloalkenylen oder ein durch Sauerstoff, Schwefel oder $\rangle$N-$R_7$ unterbrochenes unsubstituiertes $C_4$-$C_{11}$-Cycloalkenylen bilden, deren Ringe gegebenenfalls mit $C_1$-$C_4$-Alkyl, unsubstituiertem oder durch $C_1$-$C_4$-Alkyl substituiertem $C_5$-$C_{12}$-Cycloalkyl; unsubstituiertem oder mit $C_1$-$C_4$-Alkyl substituiertem Phenyl; oder mit unsubstituiertem oder mit $C_1$-$C_4$-Alkyl substituiertem Phenylen substituiert sind,

$R_7$ Wasserstoff, $C_1$-$C_8$-Alkyl, $C_1$-$C_8$-Alkoxy, $C_1$-$C_8$-Alkanoyl oder unsubstituiertes oder mit $C_1$-$C_4$-Alkyl substituiertes Benzoyl bedeutet,

$R_8$ Hydroxy,

$$\left[-O^{-}\ \frac{1}{r}M^{r+}\right]$$ , $C_1$-$C_{18}$-Alkoxy oder 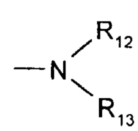

darstellt,

$R_9$ Wasserstoff, $C_1$-$C_8$-Alkanoyl oder unsubstituiertes oder mit $C_1$-$C_4$-Alkyl substituiertes Benzoyl bedeutet,

$R_{10}$ und $R_{11}$ unabhängig voneinander Wasserstoff, $CF_3$, $C_1$-$C_{12}$-Alkyl, unsubstituiertes oder durch $C_1$-$C_4$-Alkyl substituiertes Phenyl; oder -$(CH_2)_s$-$COR_{14}$ darstellen, oder $R_{10}$ und $R_{11}$ zusammen mit dem Kohlenstoffatom, an das sie gebunden sind, einen unsubstituierten oder durch 1 bis 3 $C_1$-$C_4$-Alkyl substituierten $C_5$-$C_{12}$-Cycloalkylidenring bilden,
$R_{12}$ und $R_{13}$ unabhängig voneinander Wasserstoff oder $C_1$-$C_{18}$-Alkyl darstellen,
$R_{14}$ Hydroxy, $C_1$-$C_{18}$-Alkoxy oder

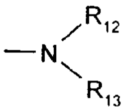

darstellt,
M ein r-wertiges Metallkation ist,
m 0, 1 oder 2 darstellt,
n 1, 2, 3, 4, 5 oder 6 bedeutet,
r 1, 2 oder 3 ist, und
s 0 oder 1 darstellt.

**23.** Verbindungen gemäss Anspruch 22, worin

$R_1$ Wasserstoff, $C_1$-$C_4$-Alkyl oder $C_7$-$C_9$-Phenylalkyl bedeutet,
$R_2$ Wasserstoff darstellt,
$R_3$ $C_1$-$C_4$-Alkyl, $C_7$-$C_9$-Phenylalkyl oder $C_1$-$C_4$-Alkoxy bedeutet,
$R_4$ Wasserstoff darstellt, und
$R_5$ und $R_6$ zusammen mit den Kohlenstoffatomen, an die sie gebunden sind, ein unsubstituiertes Cyclopentenylen oder Cycloheptenylen oder ein durch Sauerstoff oder Schwefel unterbrochenes unsubstituiertes $C_5$-Cycloalkenylen bilden, deren Ringe gegebenenfalls mit $C_1$-$C_4$-Alkyl substituiert sind.

Europäisches Patentamt

# EUROPÄISCHER RECHERCHENBERICHT

Nummer der Anmeldung
EP 97 81 0988

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | US 3 702 878 A (TORANOSUKE SAITO)<br>* das ganze Dokument * | 1-21 | C07F9/6571<br>C08K5/5393<br>C07C39/23<br>C07C39/205<br>C07D309/22<br>C07D335/02 |
| A | EP 0 000 352 A (CIBA-GEIGY AG)<br>* das ganze Dokument * | 1-21 | |
| A | EP 0 703 241 A (BAYER AG)<br>* das ganze Dokument * | 1-21 | |
| A | CHEMICAL ABSTRACTS, vol. 097, no. 20, 15.November 1982<br>Columbus, Ohio, US;<br>abstract no. 164043, "Stabilizers for polymers"<br>XP002058497<br>* Zusammenfassung *<br>& JP 57 076 079 A (SANKO KAIHATSU KAGAKU KENKYUSHO;JAPAN) 12.Mai 1982 | 1-21 | |
| | | | **RECHERCHIERTE SACHGEBIETE (Int.Cl.6)** |
| X | AKIRA NISHINAGA: "Oxygenation of tert-butylphenols with an unsaturated side chain"<br>JOURNAL OF ORGANIC CHEMISTRY.,<br>Bd. 51, Nr. 12, 1986, EASTON US,<br>Seiten 2257-2266, XP002058495<br>* Seite 2259, Verbindungen 12; Seite 2264, Kolumn 1 * | 22,23 | C07F<br>C08K<br>C07C<br>C07D |
| X | DE 10 82 273 B (FARBENFABRIKEN BAYER AG)<br>* Beispiel 4 * | 22,23 | |
| X | G. CASIRAGHI: "Selective o-vinylation of phenols; synthesis of 2-(1-phenylethenyl)-phenols"<br>SYNTHESIS.,<br>1977, STUTTGART DE,<br>Seiten 122-124, XP002058496<br>* Verbindungen der Formel 2 * | 22,23 | |

Der vorliegende Recherchenbericht wurde für alle Patentansprüche erstellt

| Recherchenort | Abschlußdatum der Recherche | Prüfer |
|---|---|---|
| DEN HAAG | 11.März 1998 | Beslier, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)